# EUROPEAN PATENT SPECIFICATION

(11) **EP 4 196 987 B1**
(45) Date of publication and mention of the grant of the patent: **23.09.2026**
(21) Application number: 21856679.2
(22) Date of filing: 11.08.2021
(51) Int. Cl.: G01N 15/075, G01N 15/06, G01N 30/00, G01N 21/33, G01N 21/41, G01N 21/51, G01N 30/78, G01N 30/86, G16B 45/00

(54) **MEASURING ATTRIBUTES OF A VIRAL GENE DELIVERY VEHICLE SAMPLE VIA SEPARATION**
MESSUNG DER EIGENSCHAFTEN EINER VIRUSGENABGABEVEHIKELPROBE DURCH TRENNUNG
MESURE D'ATTRIBUTS D'UN ÉCHANTILLON DE VÉHICULE DE DISTRIBUTION DE GÈNE VIRAL PAR L'INTERMÉDIAIRE D'UNE SÉPARATION

(30) Priority: 11.08.2020 US 202016991016
(43) Date of publication of application: 21.06.2023
(73) Proprietor: Wyatt Technology, LLC, Goleta, CA 93117 (US)
(72) Inventor: CHEN, Michelle, Goleta, California 93117 (US); PURCHEL, Anatolii, Goleta, California 93117 (US)
(74) Representative: Forresters IP LLP
(86) International application number: PCT/US2021/045625
(87) International publication number: WO 2022/036017

(56) References cited:
- WO-A1-2014/065669
- WO-A1-2020/117898
- US-A1- 2014 148 381
- US-A1- 2014 148 381
- US-A1- 2015 005 369
- US-A1- 2015 005 369

## Description

### PRIORITY

This application claims priority to U.S. Patent Application Serial No. 16/991,016, filed August 11, 2020.

### BACKGROUND

The present disclosure relates to samples, and more specifically, to measuring attributes of a viral gene delivery vehicle sample via separation.
US 2015/005369 discloses methods of gene delivery using capsid-modified recombinant adeno-associated viral (rAAV) vectors.

### SUMMARY

The present disclosure describes a computer implemented method, a system, and a computer program product of measuring attributes of a viral gene delivery vehicle (VGDV) sample via separation. The present invention is defined by the claims. In an exemplary embodiment, the computer implemented method, the system, and the computer program product include (1) executing, by a computer system, a set of logical operations analyzing a viral gene delivery vehicle (VGDV) sample on a set of analytical instruments, where the set includes at least one separation instrument, at least one static light scattering instrument, and at least two concentration detectors, resulting in a capsid protein mass of the sample, m_{A}, a modifier mass of the sample, m_{B}, and a modifier molar mass of the sample, M_{B}, (2) receiving a capsid protein molar mass of the sample, M_{A}, from a capsid protein molar mass data source, (3) receiving an injection volume of the sample, v, from an injection volume data source, and (4) executing, by the computer system, a set of logical operations calculating a total VGDV particle concentration of the sample, C_{A}, via ${C}_{A} = \left({m}_{A} x N\right) / \left({M}_{A} x v\right) ,$ where N is Avogrado's number. In an embodiment, the sample is a Lenti viral vector sample. In an embodiment, the sample is an adeno-viral vector sample. In an embodiment, the sample is an adeno-associated virus (AAV) sample. In an embodiment, the modifier mass of the sample, m_{B}, is a nucleic acid mass of the sample. In an embodiment, the modifier molar mass of the sample, MB, is a nucleic acid molar mass of the sample.

### BRIEF DESCRIPTION OF THE DRAWINGS

FIG. 1A depicts a flowchart in accordance with an exemplary embodiment.
FIG. IB depicts a block diagram in accordance with an exemplary embodiment.
FIG. 1C depicts a flowchart in accordance with an exemplary embodiment.
FIG. 1D depicts a flowchart in accordance with an exemplary embodiment.
FIG. 1E depicts a flowchart in accordance with an exemplary embodiment.
FIG. 1F depicts a flowchart in accordance with an exemplary embodiment not falling under the scope of the present invention.
FIG. 1G depicts a flowchart in accordance with an exemplary embodiment not falling under the scope of the present invention.
FIG. 2A depicts a flowchart in accordance with an embodiment.
FIG. 2B depicts a flowchart in accordance with an embodiment.
FIG. 3A depicts an apparatus in accordance with an embodiment.
FIG. 3B depicts an apparatus in accordance with an embodiment.
FIG. 3C depicts an apparatus in accordance with an embodiment.
FIG. 3D depicts an apparatus in accordance with an embodiment.
FIG. 4A depicts a graph in accordance with an embodiment.
FIG. 4B depicts a graph in accordance with an embodiment.
FIG. 4C depicts a graph in accordance with an embodiment.
FIG. 5 depicts a graph in accordance with an embodiment.
FIG. 6A depicts a graph in accordance with an embodiment.
FIG. 6B depicts a graph in accordance with an embodiment.
FIG. 6C depicts a graph in accordance with an embodiment.
FIG. 7A depicts a graph in accordance with an embodiment.
FIG. 7B depicts a graph in accordance with an embodiment.
FIG. 7C depicts a graph in accordance with an embodiment.
FIG. 8 depicts a computer system in accordance with an exemplary embodiment.

### DETAILED DESCRIPTION

The present disclosure describes a computer implemented method, a system, and a computer program product of measuring attributes of a viral gene delivery vehicle (VGDV) sample via separation. In an exemplary embodiment, the computer implemented method, the system, and the computer program product include (1) executing, by a computer system, a set of logical operations analyzing a viral gene delivery vehicle (VGDV) sample on a set of analytical instruments, where the set includes at least one separation instrument, at least one static light scattering instrument, and at least two concentration detectors, resulting in a capsid protein mass of the sample, m_{A}, a modifier mass of the sample, m_{B}, and a modifier molar mass of the sample, M_{B}, (2) receiving a capsid protein molar mass of the sample, M_{A}, from a capsid protein molar mass data source, (3) receiving an injection volume of the sample, v, from an injection volume data source, and (4) executing, by the computer system, a set of logical operations calculating a total VGDV particle concentration of the sample, C_{A}, via ${C}_{A} = \left({m}_{A} x N\right) / \left({M}_{A} x v\right) ,$ where N is Avogrado's number. In an embodiment, the sample is a Lenti viral vector sample. In an embodiment, the sample is an adeno-viral vector sample. In an embodiment, the sample is an adeno-associated virus (AAV) sample. In an embodiment, the modifier mass of the sample, m_{B}, is a nucleic acid mass of the sample. In an embodiment, the modifier molar mass of the sample, MB, is a nucleic acid molar mass of the sample.

### Definitions

### Particle

A particle may be a constituent of a liquid sample aliquot. Such particles may be molecules of varying types and sizes, nanoparticles, virus like particles, liposomes, emulsions, bacteria, and colloids. These particles may range in size on the order of nanometer to microns.

### Analysis of Macromolecular or Particle Species in Solution

The analysis of macromolecular or particle species in solution may be achieved by preparing a sample in an appropriate solvent and then injecting an aliquot thereof into a separation system such as a liquid chromatography (LC) column or field flow fractionation (FFF) channel where the different species of particles contained within the sample are separated into their various constituencies. Once separated, generally based on size, mass, or column affinity, the samples may be subjected to analysis by means of light scattering, refractive index, ultraviolet absorption, electrophoretic mobility, and viscometric response.

### Light Scattering

Light scattering (LS) is a non-invasive technique for characterizing macromolecules and a wide range of particles in solution. The two types of light scattering detection frequently used for the characterization of macromolecules are static light scattering and dynamic light scattering.

### Dynamic Light Scattering

Dynamic light scattering is also known as quasi-elastic light scattering (QELS) and photon correlation spectroscopy (PCS). In a DLS experiment, time-dependent fluctuations in the scattered light signal are measured using a fast photodetector. DLS measurements determine the diffusion coefficient of the molecules or particles, which can in turn be used to calculate their hydrodynamic radius.

### Static Light Scattering

Static light scattering (SLS) includes a variety of techniques, such as single angle light scattering (SALS), dual angle light scattering (DALS), low angle light scattering (LALS), and multi-angle light scattering (MALS). SLS experiments generally involve the measurement of the absolute intensity of the light scattered from a sample in solution that is illuminated by a fine beam of light. Such measurement is often used, for appropriate classes of particles/molecules, to determine the size and structure of the sample molecules or particles, and, when combined with knowledge of the sample concentration, the determination of weight average molar mass. In addition, nonlinearity of the intensity of scattered light as a function of sample concentration may be used to measure interparticle interactions and associations.

### Multi-Angle Light Scattering

Multi-angle light scattering (MALS) is a SLS technique for measuring the light scattered by a sample into a plurality of angles. It is used for determining both the absolute molar mass and the average size of molecules in solution, by detecting how they scatter light. Collimated light from a laser source is most often used, in which case the technique can be referred to as multiangle laser light scattering (MALLS). The "multi-angle" term refers to the detection of scattered light at different discrete angles as measured, for example, by a single detector moved over a range that includes the particular angles selected or an array of detectors fixed at specific angular locations.

A MALS measurement requires a set of ancillary elements. Most important among them is a collimated or focused light beam (usually from a laser source producing a collimated beam of monochromatic light) that illuminates a region of the sample. The beam is generally plane-polarized perpendicular to the plane of measurement, though other polarizations may be used especially when studying anisotropic particles. Another required element is an optical cell to hold the sample being measured. Alternatively, cells incorporating means to permit measurement of flowing samples may be employed. If single-particles scattering properties are to be measured, a means to introduce such particles one-at-a-time through the light beam at a point generally equidistant from the surrounding detectors must be provided.

Although most MALS-based measurements are performed in a plane containing a set of detectors usually equidistantly placed from a centrally located sample through which the illuminating beam passes, three-dimensional versions also have been developed where the detectors lie on the surface of a sphere with the sample controlled to pass through its center where it intersects the path of the incident light beam passing along a diameter of the sphere. The MALS technique generally collects multiplexed data sequentially from the outputs of a set of discrete detectors. The MALS light scattering photometer generally has a plurality of detectors.

Normalizing the signals captured by the photodetectors of a MALS detector at each angle may be necessary because different detectors in the MALS detector (i) may have slightly different quantum efficiencies and different gains, and (ii) may look at different geometrical scattering volumes. Without normalizing for these differences, the MALS detector results could be nonsensical and improperly weighted toward different detector angles.

### Concentration Detector

### Differential Refractive Index Detector

A differential refractive index detector (dRI), or differential refractometer, or refractive index detector (RI or RID), is a detector that measures the refractive index of an analyte relative to the solvent. They are often used as detectors for high-performance liquid chromatography and size exclusion chromatography. dRIs are considered to be universal detectors because they can detect anything with a refractive index different from the solvent, but they have low sensitivity. When light leaves one material and enters another it bends, or refracts. The refractive index of a material is a measure of how much light bends when it enters.

A differential refractive index detector contain a flow cell with the following two parts: one for the sample; and one for the reference solvent. The dRI measures the refractive index of both components. When only solvent is passing through the sample component, the measured refractive index of both components is the same, but when an analyte passes through the flow cell, the two measured refractive indices are different. The difference appears as a peak in the chromatogram. Differential refractive index detectors are often used for the analysis of polymer samples in size exclusion chromatography. A dRI could output a concentration detector signal value corresponding to a concentration value of a sample.

### Ultraviolet-visible Spectroscopy

Ultraviolet-visible spectroscopy or ultraviolet-visible spectrophotometry (UV-Vis or UV/Vis) refers to absorption spectroscopy or reflectance spectroscopy in the ultraviolet-visible spectral region. An ultraviolet-visible detector/ultraviolet-visible spectrophotometer uses light in the visible and adjacent ranges, where the absorption or reflectance in the visible range directly affects the perceived color of the chemicals involved, where in this region of the electromagnetic spectrum, atoms and molecules undergo electronic transitions. Such absorption spectroscopy measures transitions from the ground state to the excited state. An ultraviolet-visible detector/ultraviolet-visible spectrophotometer measures the intensity of light passing through a sample (I), and compares it to the intensity of light before it passes through the sample (Iₒ), where the ratio I/Iₒ is called the transmittance, and is usually expressed as a percentage (%T). The absorbance, A, is based on the transmittance according to $A = - log \left(%T/100%\right) .$ The UV-visible spectrophotometer can also be configured to measure reflectance, where the spectrophotometer measures the intensity of light reflected from a sample (I), and compares it to the intensity of light reflected from a reference material (Iₒ), where the ratio I/Iₒ is called the reflectance, and is usually expressed as a percentage (%R). An ultraviolet absorption detector could output a concentration detector signal value corresponding to a concentration value of a sample.

### Adeno-Associated Virus

Adeno-associated virus (AAV) is a small virus (~20 nm) from a family of Parvoviridae that infects humans but is believed to not cause any disease. Because of its small size, mild immune response, and the ability to stably integrate its genome into a host cell genome at a specific site (AAVS1 on human chromosome 19) AAV emerged as an attractive vector for gene therapy. With the recent FDA approval of Zolgesma^{®} to treat spinal muscular dystrophy and several promising clinical trials underway, such as trial number NCT00516477 (Clinicaltrials.gov), AAV manufacturing process requires robust, reliable, and easy-to-implement characterization methods to meet regulatory requirements imposed by FDA and other regulatory agencies. However, the characterization of viral vectors remains a challenge and new methods have to be developed to assure safe and high-quality AAV vectors for the advancement of AAV-related clinical studies. Additionally, FDA recently developed two reference standard materials (RSMs) - recombinant AAV serotypes 2 and 8 which can be used as benchmarking tools for demonstrating that the characterization methods are appropriately controlled and for qualifying in-house reference materials. When these RSMs were established, great variability was noticed in the determination of capsid particles and vector genomes between different institutions which further emphasizes the need in robust methods to determine titers of AAV vectors in preclinical and clinical studies when the field is developing so rapidly.

AAV characterization is usually separated in a few different stages: particle titer, vector genome titer, transducing titer, infectious titer, and determination of purity and identity. Particle titer quantification usually involves ELISA assay (enzyme-linked immunosorbent assay) to detect the presence of a ligand (protein) in a solution using antibodies directed against the protein to be measured. Because this test requires a special laboratory to be performed in, obtaining results usually takes from 24 hours to several weeks. Next step is to quantify the amount of viral genome. This is usually done using qPCR (quantitative polymerase chain reaction) after lysis of the viral capsid and thus, loss of a sample.

Purity and identity of the obtained AAVs is evaluated by SDS-PAGE (sodium dodecyl sulfate-polyacrylamide gel electrophoresis). Obtained viral capsid protein bands are evaluated for their stoichiometry and size. But this is a relative method that requires the use of standards. Gene vector identity was determined by observing the electrophoretic banding pattern and comparing it to a positive control (also a relative technique).

Therefore, it is critical to have a robust and reproducible method that can be easily implemented in QC during manufacturing. SEC-MALS allows rapid sample analysis with run times under 30 minutes and can be used to determine critical quality attributes of AAV-based gene therapy agents such as number concentration of viral capsids, the ratio of filled vs unfilled particles, absolute molar mass of protein and genome components. There is a need to measure attributes of a viral gene delivery vehicle (VGDV) sample via separation.

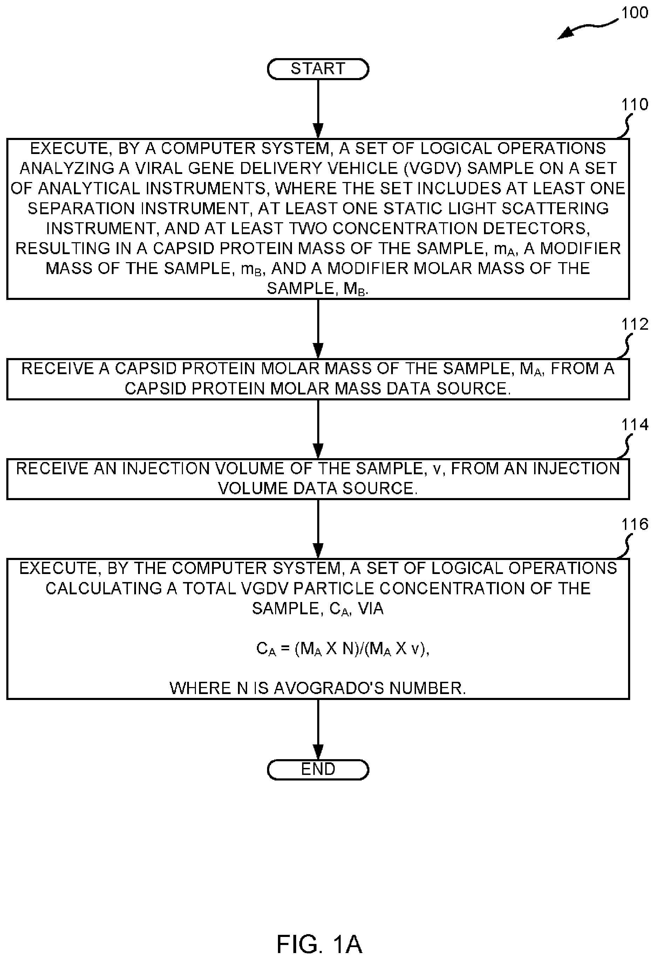

Referring to FIG. 1A, in an exemplary embodiment, the computer implemented method, the system, and the computer program product are configured to perform an operation 110 of executing, by a computer system, a set of logical operations analyzing a viral gene delivery vehicle (VGDV) sample on a set of analytical instruments, where the set includes at least one separation instrument, at least one static light scattering instrument, and at least two concentration detectors, resulting in a capsid protein mass of the sample, m_{A}, a modifier mass of the sample, m_{B}, and a modifier molar mass of the sample, M_{B}, an operation 112 of receiving a capsid protein molar mass of the sample, M_{A}, from a capsid protein molar mass data source, an operation 114 of receiving an injection volume of the sample, v, from an injection volume data source, and an operation 116 of executing, by the computer system, a set of logical operations calculating a total VGDV particle concentration of the sample, C_{A}, via ${C}_{A} = \left({m}_{A} x N\right) / \left({M}_{A} x v\right) ,$ where N is Avogrado's number.

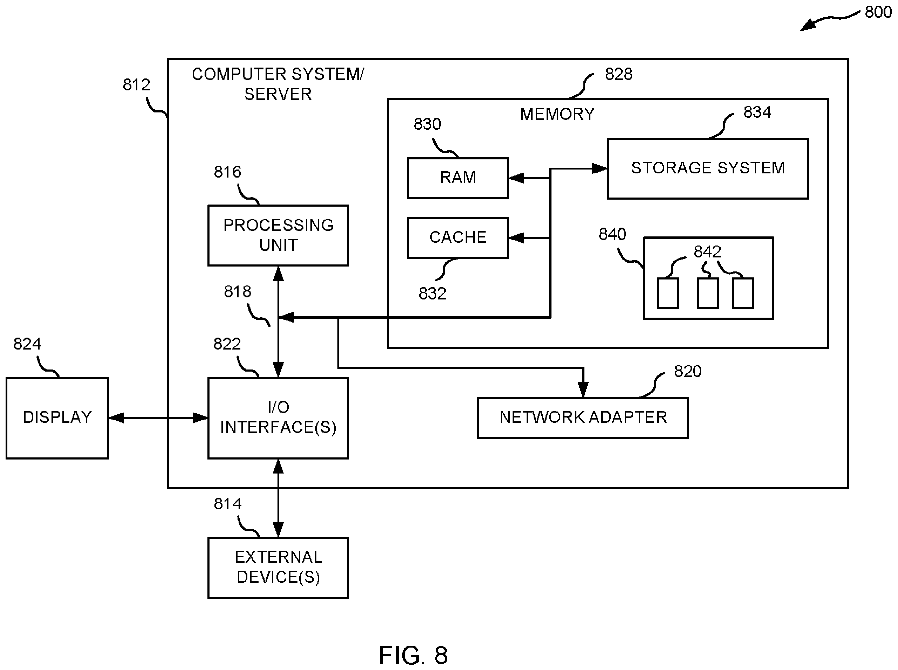

In an exemplary embodiment, the computer system is a standalone computer system, such as computer system 800 shown in FIG. 8, a network of distributed computers, where at least some of the computers are computer systems such as computer system 800 shown in FIG. 8, or a cloud computing node server, such as computer system 800 shown in FIG. 8. In an embodiment, the computer system is a computer system 800 as shown in FIG. 8, that executes a measuring attributes of a viral gene delivery vehicle (VGDV) sample via separation script or computer software application that carries out the operations of at least method 100. In an embodiment, the computer system is a computer system/server 812 as shown in FIG. 8, that executes a measuring attributes of a viral gene delivery vehicle (VGDV) sample via separation script or computer software application that carries out the operations of at least method 100. In an embodiment, the computer system is a processing unit 816 as shown in FIG. 8, that executes a measuring attributes of a viral gene delivery vehicle (VGDV) sample via separation script or computer software application that carries out the operations of at least method 100. In an embodiment, the computer system is a processor of the analytical instrument, that executes a measuring attributes of a viral gene delivery vehicle (VGDV) sample via separation script or computer software application that carries out the operations of at least method 100.

In an embodiment, the computer system is a computer system 800 as shown in FIG. 8, that executes a measuring attributes of a viral gene delivery vehicle (VGDV) sample via separation script or computer software application that carries out at least operations 110, 112, 114, and 116. In an embodiment, the computer system is a computer system/server 812 as shown in FIG. 8, that executes a measuring attributes of a viral gene delivery vehicle (VGDV) sample via separation script or computer software application that carries out at least operations 110, 112, 114, and 116. In an embodiment, the computer system is a processing unit 816 as shown in FIG. 8, that executes a measuring attributes of a viral gene delivery vehicle (VGDV) sample via separation script or computer software application that carries out at least operations 110, 112, 114, and 116.

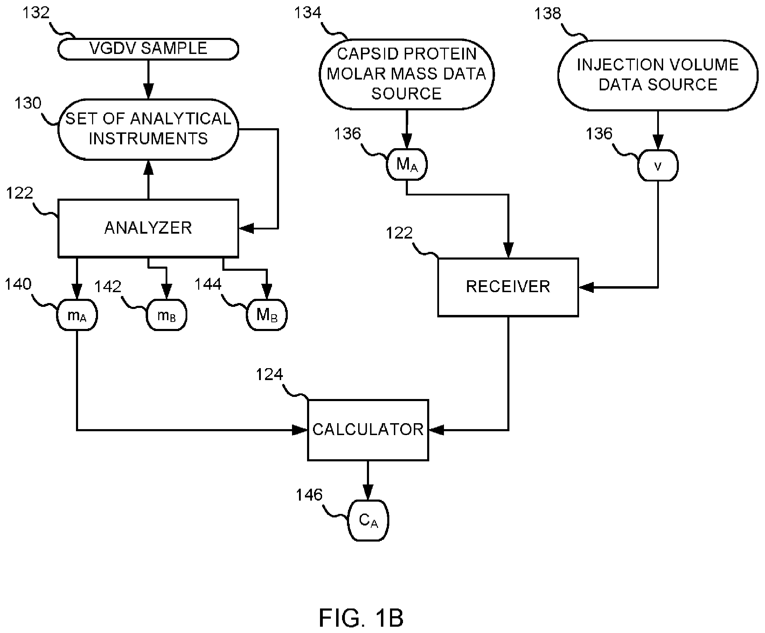

Referring to FIG. 1B, in an exemplary embodiment, the computer implemented method, the system, and the computer program product include an analyzer 120, a receiver 122, and a calculator 124. In an embodiment, analyzer 120 is configured to execute a set of logical operations analyzing a viral gene delivery vehicle (VGDV) sample 132 on a set of analytical instruments 130, where the set includes at least one separation instrument, at least one static light scattering instrument, and at least two concentration detectors, resulting in a capsid protein mass of the sample 140, m_{A}, a modifier mass of the sample 142, m_{B}, and a modifier molar mass of the sample 144, M_{B}. In an embodiment, analyzer 120 includes a computer system, such as computer system 800 as shown in FIG. 8, performing operation 110. In an embodiment, analyzer 120 includes a computer system, such as computer system/server 812 as shown in FIG. 8, performing operation 110. In an embodiment, analyzer 120 includes a computer system, such as processing unit 816 as shown in FIG. 8, performing operation 110. In an embodiment, analyzer 120 is implemented as computer software executing on a computer system, such as computer system 800 as shown in FIG. 8, such that the computer system performs operation 110. In an embodiment, analyzer 120 is implemented as computer software executing on a computer system, such as computer system/server 812 as shown in FIG. 8, such that the computer system performs operation 110. In an embodiment, analyzer 120 is implemented as computer software executing on a computer system, such as processing unit 816 as shown in FIG. 8, such that the computer system performs operation 110. In an embodiment, analyzer 120 performs operation 110 as computer software executing on a processor of analyzer 120.

In an embodiment, receiver 122 is configured to receive a capsid protein molar mass of the sample 136, M_{A}, from a capsid protein molar mass data source 134. In an embodiment, receiver 122 includes a computer system, such as computer system 800 as shown in FIG. 8, performing operation 112. In an embodiment, receiver 122 includes a computer system, such as computer system/server 812 as shown in FIG. 8, performing operation 112. In an embodiment, receiver 122 includes a computer system, such as processing unit 816 as shown in FIG. 8, performing operation 112. In an embodiment, receiver 122 is implemented as computer software executing on a computer system, such as computer system 800 as shown in FIG. 8, such that the computer system performs operation 112. In an embodiment, receiver 122 is implemented as computer software executing on a computer system, such as computer system/server 812 as shown in FIG. 8, such that the computer system performs operation 112. In an embodiment, receiver 122 is implemented as computer software executing on a computer system, such as processing unit 816 as shown in FIG. 8, such that the computer system performs operation 112. In an embodiment, receiver 122 performs operation 112 as computer software executing on a processor of receiver 122.

In an embodiment, receiver 122 is configured to receive an injection volume of the sample 139, v, from an injection volume data source 138. In an embodiment, receiver 122 includes a computer system, such as computer system 800 as shown in FIG. 8, performing operation 114. In an embodiment, receiver 122 includes a computer system, such as computer system/server 812 as shown in FIG. 8, performing operation 114. In an embodiment, receiver 122 includes a computer system, such as processing unit 816 as shown in FIG. 8, performing operation 114. In an embodiment, receiver 122 is implemented as computer software executing on a computer system, such as computer system 800 as shown in FIG. 8, such that the computer system performs operation 114. In an embodiment, receiver 122 is implemented as computer software executing on a computer system, such as computer system/server 812 as shown in FIG. 8, such that the computer system performs operation 114. In an embodiment, receiver 122 is implemented as computer software executing on a computer system, such as processing unit 816 as shown in FIG. 8, such that the computer system performs operation 114. In an embodiment, receiver 122 performs operation 114 as computer software executing on a processor of receiver 122.

In an embodiment, calculator 124 is configured to execute a set of logical operations calculating a total VGDV particle concentration of the sample 146, C_{A}, via ${C}_{A} = \left({m}_{A} x N\right) / \left({M}_{A} x v\right) ,$ where N is Avogrado's number. In an embodiment, calculator 124 includes a computer system, such as computer system 800 as shown in FIG. 8, performing operation 116. In an embodiment, calculator 124 includes a computer system, such as computer system/server 812 as shown in FIG. 8, performing operation 116. In an embodiment, calculator 124 includes a computer system, such as processing unit 816 as shown in FIG. 8, performing operation 116. In an embodiment, calculator 124 is implemented as computer software executing on a computer system, such as computer system 800 as shown in FIG. 8, such that the computer system performs operation 116. In an embodiment, calculator 124 is implemented as computer software executing on a computer system, such as computer system/server 812 as shown in FIG. 8, such that the computer system performs operation 116. In an embodiment, calculator 124 is implemented as computer software executing on a computer system, such as processing unit 816 as shown in FIG. 8, such that the computer system performs operation 116. In an embodiment, calculator 124 performs operation 116 as computer software executing on a processor of calculator 124.

### Instruments

### Separation Instruments

In an embodiment, the at least one separation instrument includes at least one of a size exclusion chromatography (SEC) unit, a field flow fractionation (FFF) unit, and an ion-exchange chromatography (IEX) unit. In an embodiment, the at least one separation instrument is at least one of a SEC unit, a FFF unit, and an IEX unit.

### Static Light Scattering Instrument

In an embodiment, the at least one static light scattering (SLS) instrument includes a multi-angle light scattering (MALS) instrument. In an embodiment, the at least one static light scattering (SLS) instrument is a MALS instrument.

### Concentration Detectors

### UV-UV

In an embodiment, the at least two concentration detectors include a first ultra-violet absorbance (UV) detector at a first wavelength, λ1, and a second ultra-violet absorbance (UV) detector at a second wavelength, λ2. In an embodiment, the at least two concentration detectors are a first UV detector at a first wavelength, λ1, and a second UV detector at a second wavelength, λ2. In a particular embodiment, the first wavelength, λ1, is 260 nm, and the second wavelength, λ2, is 280 nm.

### UV-dRI

In an embodiment, the at least two concentration detectors include an ultra-violet absorbance (UV) detector at a wavelength, λ, and a differential refractive index (dRI) detector. In an embodiment, the at least two concentration detectors are a UV detector at a wavelength, λ, and a dRI detector. In a particular embodiment, the wavelength, λ, is one of 260 nm and 280 nm.

### UV-FLD

In an embodiment, the at least two concentration detectors include an ultra-violet absorbance (UV) detector at a wavelength, λ, and a fluorescence detector (FLD). In an embodiment, the at least two concentration detectors are a UV detector at a wavelength, λ, and a FLD. In a particular embodiment, the wavelength, λ, is one of 260 nm and 280 nm.

### dRI-FLD

In an embodiment, the at least two concentration detectors include a differential refractive index (dRI) detector and a fluorescence detector (FLD). In an embodiment, the at least two concentration detectors are a dRI detector and a FLD.

### Choice of Detectors

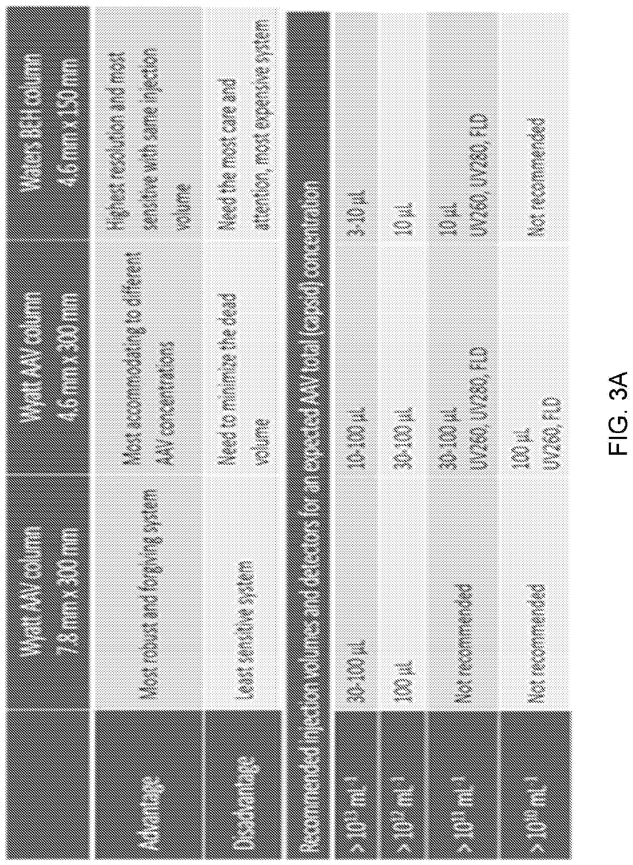

The use of different concentration detectors may depend on starting sample quality, concentration, and a total volume available for the analysis. Sensitivity of the method depends on the factors that are summarized in the table in FIG. 3A.

In general, each combination of concentration detectors could be used as follows:
dRI-FLD - used only if the sample has a fluorescent tag with known excitation and emission wavelengths;
UV-FLD - for sample concentrations ~1010 particles/mL;
UV-UV - for sample concentrations ~1011 particles/mL; and
UV-dRI - for sample concentrations above 1012 particles/mL.

### Set of Instruments

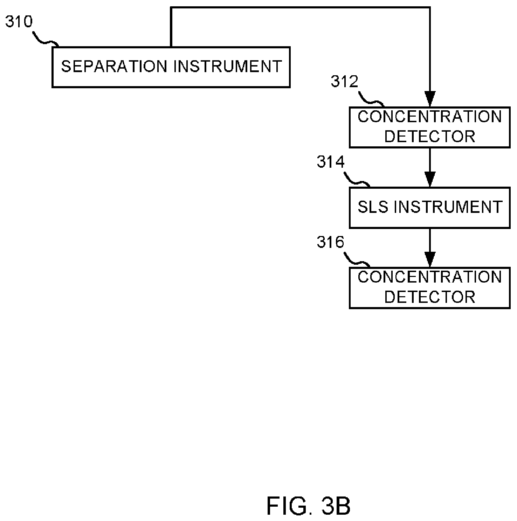

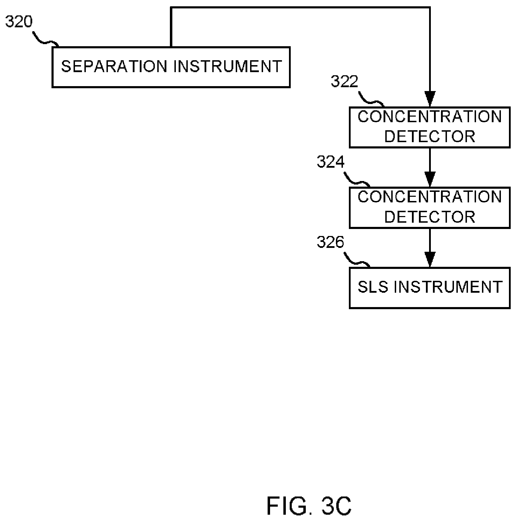

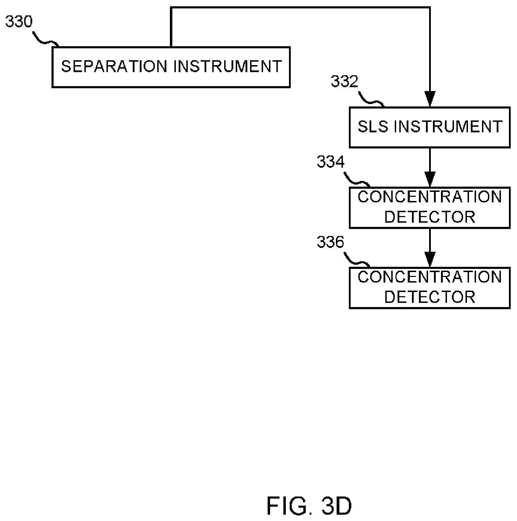

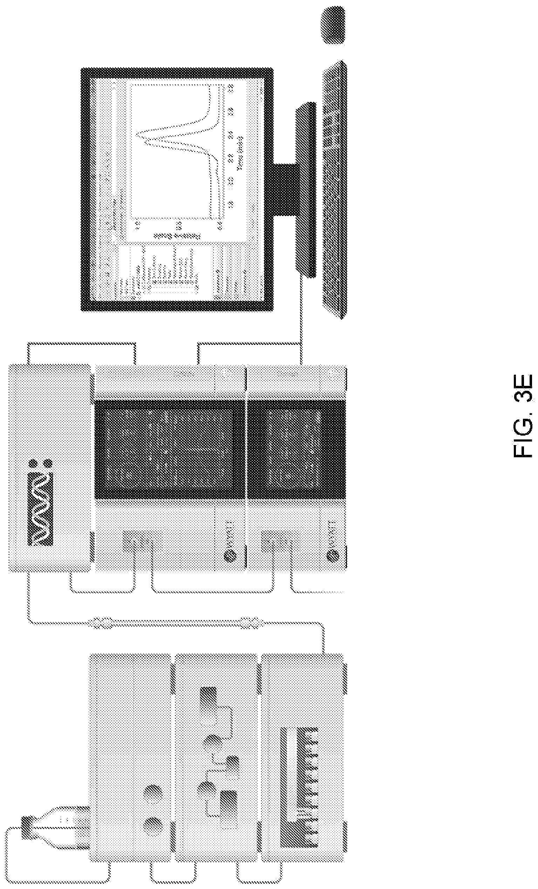

Referring to FIG. 3B, in an embodiment, at least one separation instrument 310 is connected to a first concentration detector 312, where first concentration detector 312 is connected to at least one SLS instrument 314, where at least one SLS instrument 314 is connected to a second concentration detector 316. Referring to FIG. 3C, in an embodiment, at least one separation instrument 320 is connected to a first concentration detector 322, where first concentration detector 322 is connected to a second concentration detector 324, where second concentration detector 324 is connected to at least one SLS instrument 326. Referring to FIG. 3D, in an embodiment, at least one separation instrument 330 is connected to at least one SLS instrument 332, where at least one SLS instrument 332 is connected to a first concentration detector 334, where first concentration detector 334 is connected to a second concentration detector 336. For example, FIG. 3E depicts a typical set of instruments.

### Calculating Capsid Protein Molar Mass

In a further embodiment, the computer implemented method, the system, and the computer program product further include (a) executing, by the computer system, a set of logical operations analyzing the sample on the set, resulting in the capsid protein molar mass of the sample, M_{A}, and (b) storing the capsid protein molar mass of the sample, M_{A}, in the capsid protein molar mass data source. In a further embodiment, the method, the system, and the computer program product further include (a) executing, by the computer system, a set of logical operations analyzing the sample on set 130, resulting in capsid protein molar mass of the sample 136, M_{A}, and (b) storing capsid protein molar mass of the sample 136, M_{A}, in capsid protein molar mass data source 134.

### Analyzing Sample

In an embodiment, the analyzing includes analyzing the sample on the set via an analysis technique, where the analysis technique is one of viral vector analysis, protein conjugate analysis, and copolymer composition analysis. In an embodiment, analyzing operation 110 includes an operation of analyzing sample 132 on set 130 via an analysis technique, where the analysis technique is one of viral vector analysis, protein conjugate analysis, and copolymer composition analysis.

### VGDV Concentration

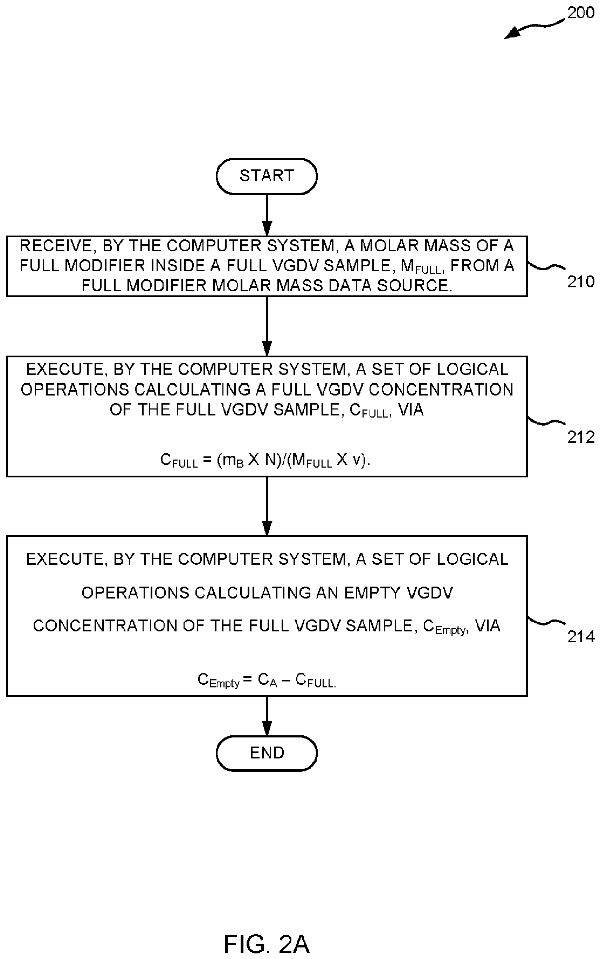

In a further embodiment, the computer implemented method, the system, and the computer program product further include (a) receiving, by the computer system, a molar mass of a full modifier inside a full VGDV sample, M_{Full}, from a full modifier molar mass data source, (b) executing, by the computer system, a set of logical operations calculating a full VGDV concentration of the full VGDV sample, C_{Full}, via ${C}_{Full} = \left({m}_{B} x N\right) / \left({M}_{Full} x v\right) ,$ and
(c) executing, by the computer system, a set of logical operations calculating an empty VGDV concentration of the full VGDV sample, C_{Empty}, via ${C}_{Empty} = {C}_{A} - {C}_{Full} .$ Referring to FIG. 2A, the computer implemented method, the system, and the computer program product are further configured to perform an operation 210 of receiving, by the computer system, a molar mass of a full modifier inside a full VGDV sample, M_{Full}, from a full modifier molar mass data source, an operation 212 of executing, by the computer system, a set of logical operations calculating a full VGDV concentration of the full VGDV sample, C_{Full}, via ${C}_{Full} = \left({m}_{B} x N\right) / \left({M}_{Full} x v\right) ,$ and an operation 214 of executing, by the computer system, a set of logical operations calculating an empty VGDV concentration of the full VGDV sample, C_{Empty}, via ${C}_{Empty} = {C}_{A} - {C}_{Full} .$

In an embodiment, the computer system is a computer system 800 as shown in FIG. 8, that executes a measuring attributes of a viral gene delivery vehicle (VGDV) sample via separation script or computer software application that carries out the operations of at least method 200. In an embodiment, the computer system is a computer system/server 812 as shown in FIG. 8, that executes a measuring attributes of a viral gene delivery vehicle (VGDV) sample via separation script or computer software application that carries out the operations of at least method 200. In an embodiment, the computer system is a processing unit 816 as shown in FIG. 8, that executes a measuring attributes of a viral gene delivery vehicle (VGDV) sample via separation script or computer software application that carries out the operations of at least method 200. In an embodiment, the computer system is a processor of the analytical instrument, that executes a measuring attributes of a viral gene delivery vehicle (VGDV) sample via separation script or computer software application that carries out the operations of at least method 200.

In an embodiment, the computer system is a computer system 800 as shown in FIG. 8, that executes a measuring attributes of a viral gene delivery vehicle (VGDV) sample via separation script or computer software application that carries out at least operations 210, 212, and 214. In an embodiment, the computer system is a computer system/server 812 as shown in FIG. 8, that executes a measuring attributes of a viral gene delivery vehicle (VGDV) sample via separation script or computer software application that carries out at least operations 210, 212, and 214. In an embodiment, the computer system is a processing unit 816 as shown in FIG. 8, that executes a measuring attributes of a viral gene delivery vehicle (VGDV) sample via separation script or computer software application that carries out at least operations 210, 212, and 214.

In a further embodiment, the method, the system, and the computer program product further include (a) executing, by the computer system, a set of logical operations analyzing the full VGDV sample on the set, resulting in the molar mass of the full modifier inside the full VGDV sample, M_{Full}, and (b) storing the molar mass of the full modifier inside the full VGDV sample, M_{Full}, in the full modifier molar mass data source. In a further embodiment, the method, the system, and the computer program product further include (a) executing, by the computer system, a set of logical operations analyzing the full VGDV sample on set 130, resulting in the molar mass of the full modifier inside the full VGDV sample, M_{Full}, and (b) storing the molar mass of the full modifier inside the full VGDV sample, M_{Full}, in the full modifier molar mass data source. In an embodiment, the modifier is a nucleic acid.

### Total VGDV Peak Particle Concentration

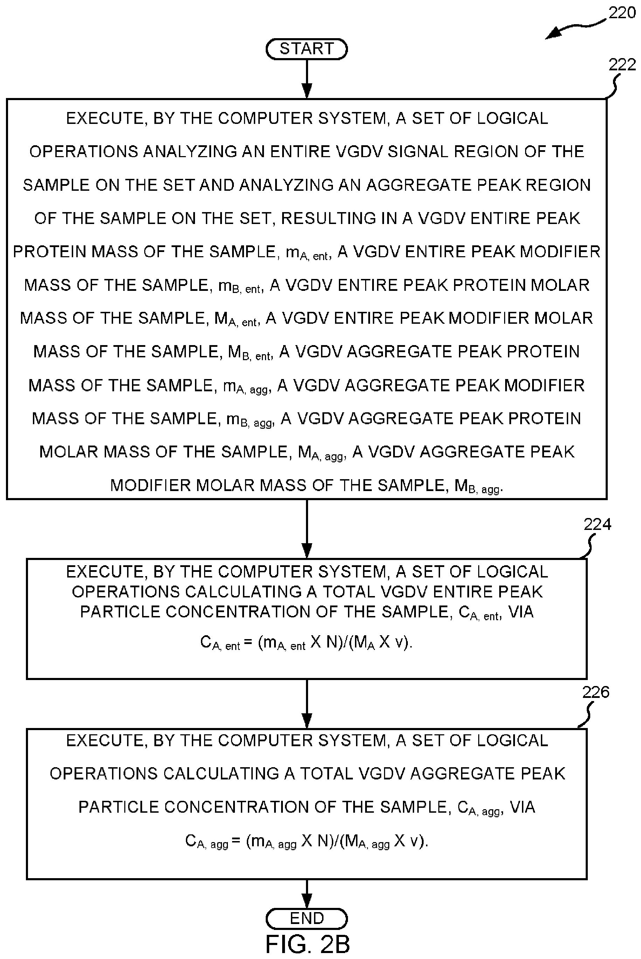

In a further embodiment, the method, the system, and the computer program product further include (a) executing, by the computer system, a set of logical operations analyzing an entire VGDV signal region of the sample on the set and analyzing an aggregate peak region of the sample on the set, resulting in a VGDV entire peak protein mass of the sample, m_{A, ent}, corresponding to the entire VGDV signal region of the sample, a VGDV entire peak modifier mass of the sample, m_{B, ent}, corresponding to the entire VGDV signal region of the sample, a VGDV entire peak protein molar mass of the sample, M_{A, ent}, corresponding to the entire VGDV signal region of the sample, a VGDV entire peak modifier molar mass of the sample, M_{B, ent}, corresponding to the entire VGDV signal region of the sample, a VGDV aggregate peak protein mass of the sample, m_{A, agg}, corresponding to the aggregate peak region of the sample, a VGDV aggregate peak modifier mass of the sample, m_{B, agg}, corresponding to the aggregate peak region of the sample, a VGDV aggregate peak protein molar mass of the sample, M_{A, agg}, corresponding to the aggregate peak region of the sample, a VGDV aggregate peak modifier molar mass of the sample, M_{B, agg}, corresponding to the aggregate peak region of the sample, (b) executing, by the computer system, a set of logical operations calculating a total VGDV entire peak particle concentration of the sample, C_{A, ent}, corresponding to the entire VGDV signal region of the sample, via ${C}_{A , ent} = \left({m}_{A , ent} x N\right) / \left({M}_{A , ent} x v\right) ,$ and
(c) executing, by the computer system, a set of logical operations calculating a total VGDV aggregate peak particle concentration of the sample, C_{A, agg}, corresponding to the aggregate peak region of the sample, via ${C}_{A , agg} = \left({m}_{A , agg} x N\right) / \left({M}_{A , agg} x v\right) .$ Referring to FIG. 2B, the computer implemented method, the system, and the computer program product are further configured to perform an operation 222 of executing, by the computer system, a set of logical operations analyzing an entire VGDV signal region of the sample on the set and analyzing an aggregate peak region of the sample on the set, resulting in a VGDV entire peak protein mass of the sample, m_{A, ent}, corresponding to the entire VGDV signal region of the sample, a VGDV entire peak modifier mass of the sample, m_{B, ent}, corresponding to the entire VGDV signal region of the sample, a VGDV entire peak protein molar mass of the sample, M_{A, ent}, corresponding to the entire VGDV signal region of the sample, a VGDV entire peak modifier molar mass of the sample, M_{B, ent}, corresponding to the entire VGDV signal region of the sample, a VGDV aggregate peak protein mass of the sample, m_{A, agg}, corresponding to the aggregate peak region of the sample, a VGDV aggregate peak modifier mass of the sample, m_{B, agg}, corresponding to the aggregate peak region of the sample, a VGDV aggregate peak protein molar mass of the sample, M_{A, agg}, corresponding to the aggregate peak region of the sample, a VGDV aggregate peak modifier molar mass of the sample, M_{B, agg}, corresponding to the aggregate peak region of the sample, an operation 224 of executing, by the computer system, a set of logical operations calculating a total VGDV entire peak particle concentration of the sample, C_{A, ent}, corresponding to the entire VGDV signal region of the sample, via ${C}_{A , ent} = \left({m}_{A , ent} x N\right) / \left({M}_{A , ent} x v\right) ,$ and
an operation 226 of executing, by the computer system, a set of logical operations calculating a total VGDV aggregate peak particle concentration of the sample, C_{A, agg}, corresponding to the aggregate peak region of the sample, via ${C}_{A , agg} = \left({m}_{A , agg} x N\right) / \left({M}_{A , agg} x v\right) .$

In an embodiment, the computer system is a computer system 800 as shown in FIG. 8, that executes a measuring attributes of a viral gene delivery vehicle (VGDV) sample via separation script or computer software application that carries out the operations of at least method 220. In an embodiment, the computer system is a computer system/server 812 as shown in FIG. 8, that executes a measuring attributes of a viral gene delivery vehicle (VGDV) sample via separation script or computer software application that carries out the operations of at least method 220. In an embodiment, the computer system is a processing unit 816 as shown in FIG. 8, that executes a measuring attributes of a viral gene delivery vehicle (VGDV) sample via separation script or computer software application that carries out the operations of at least method 220. In an embodiment, the computer system is a processor of the analytical instrument, that executes a measuring attributes of a viral gene delivery vehicle (VGDV) sample via separation script or computer software application that carries out the operations of at least method 220.

In an embodiment, the computer system is a computer system 800 as shown in FIG. 8, that executes a measuring attributes of a viral gene delivery vehicle (VGDV) sample via separation script or computer software application that carries out at least operations 222, 224, and 226. In an embodiment, the computer system is a computer system/server 812 as shown in FIG. 8, that executes a measuring attributes of a viral gene delivery vehicle (VGDV) sample via separation script or computer software application that carries out at least operations 222, 224, and 226. In an embodiment, the computer system is a processing unit 816 as shown in FIG. 8, that executes a measuring attributes of a viral gene delivery vehicle (VGDV) sample via separation script or computer software application that carries out at least operations 222, 224, and 226.

In a further embodiment, the method, the system, and the computer program product further include (a) executing, by the computer system, a set of logical operations analyzing an entire VGDV signal region of a full viral gene delivery vehicle sample on the set and analyzing an aggregate peak region of the full sample on the set, resulting in a VGDV entire peak molar mass of a full modifier inside the full sample, M_{Full, ent}, corresponding to the entire VGDV signal region of the full sample, and a VGDV aggregate peak molar mass of the full modifier inside the full sample, M_{Full, agg}, corresponding to the aggregate peak region of the full sample, (b) executing, by the computer system, a set of logical operations calculating a VGDV entire peak full VGDV concentration of the full sample, C_{Full, ent}, corresponding to the entire VGDV signal region of the VGDV sample, via ${C}_{Full , ent} = \left({m}_{B , ent} x N\right) / \left({M}_{Full , ent} x v\right) ,$
(c) executing, by the computer system, a set of logical operations calculating a VGDV aggregate peak full VGDV concentration of the full sample, C_{Full, agg}, corresponding to the aggregate peak region of the full sample, via ${C}_{Full , agg} = \left({m}_{B , agg} x N\right) / \left({M}_{Full , agg} x v\right) ,$
(d) executing, by the computer system, a set of logical operations calculating a VGDV entire peak empty VGDV concentration of the full sample, C_{Empty, ent}, corresponding to the entire VGDV signal region of the full sample, via ${C}_{Empty , ent} = {C}_{A , ent} - {C}_{Full , ent} ,$ and
(e) executing, by the computer system, a set of logical operations calculating a VGDV aggregate peak empty VGDV concentration of the full sample, C_{Empty, agg}, corresponding to the aggregate peak region of the full sample, via ${C}_{Empty , agg} = {C}_{A , agg} - {C}_{Full , agg} .$
In a further embodiment, the method, the system, and the computer program product further include (a) executing, by the computer system, a set of logical operations analyzing an entire VGDV signal region of a full viral gene delivery vehicle sample on set 130 and analyzing an aggregate peak region of the full sample on set 130, resulting in a VGDV entire peak molar mass of a full modifier inside the full sample, M_{Full, ent}, corresponding to the entire VGDV signal region of the full sample, and a VGDV aggregate peak molar mass of the full modifier inside the full sample, M_{Full, agg}, corresponding to the aggregate peak region of the full sample, (b) executing, by the computer system, a set of logical operations calculating a VGDV entire peak full VGDV concentration of the full sample, C_{Full, ent}, corresponding to the entire VGDV signal region of the VGDV sample, via ${C}_{Full , ent} = \left({m}_{B , ent} x N\right) / \left({M}_{Full , ent} x v\right) ,$
(c) executing, by the computer system, a set of logical operations calculating a VGDV aggregate peak full VGDV concentration of the full sample, C_{Full, agg}, corresponding to the aggregate peak region of the full sample, via ${C}_{Full , agg} = \left({m}_{B , agg} x N\right) / \left({M}_{Full , agg} x v\right) ,$
(d) executing, by the computer system, a set of logical operations calculating a VGDV entire peak empty VGDV concentration of the full sample, C_{Empty, ent}, corresponding to the entire VGDV signal region of the full sample, via ${C}_{Empty , ent} = {C}_{A , ent} - {C}_{Full , ent} ,$ and
(e) executing, by the computer system, a set of logical operations calculating a VGDV aggregate peak empty VGDV concentration of the full sample, C_{Empty, agg}, corresponding to the aggregate peak region of the full sample, via ${C}_{Empty , agg} = {C}_{A , agg} - {C}_{Full , agg} .$

### Approximating Total VGDV Particle Concentration

In an exemplary embodiment, the computer implemented method, the system, and the computer program product include (1) executing, by a computer system, a set of logical operations analyzing a viral gene delivery vehicle (VGDV) sample on a set of analytical instruments, where the set includes at least one separation instrument, at least one static light scattering (SLS) instrument, and at least one concentration detector, resulting in a modifier mass of the sample, m_{B}, a modifier molar mass of the sample, M_{B}, and at least one UV extinction coefficient of the sample, (2) executing, by the computer system, a set of logical operations calculating a capsid protein mass of the sample, m_{A}, and a capsid protein molar mass of the sample, M_{A}, with respect to at least one refractive-index increment value from the at least one concentration detector, (3) receiving an injection volume of the sample, v, from an injection volume data source, and (4) executing, by the computer system, a set of logical operations calculating a total VGDV particle concentration of the sample, C_{A}, via ${C}_{A} = \left({m}_{A} x N\right) / \left({M}_{A} x v\right) ,$ where N is Avogrado's number. In an embodiment, the at least one concentration detector is a dRI detector.

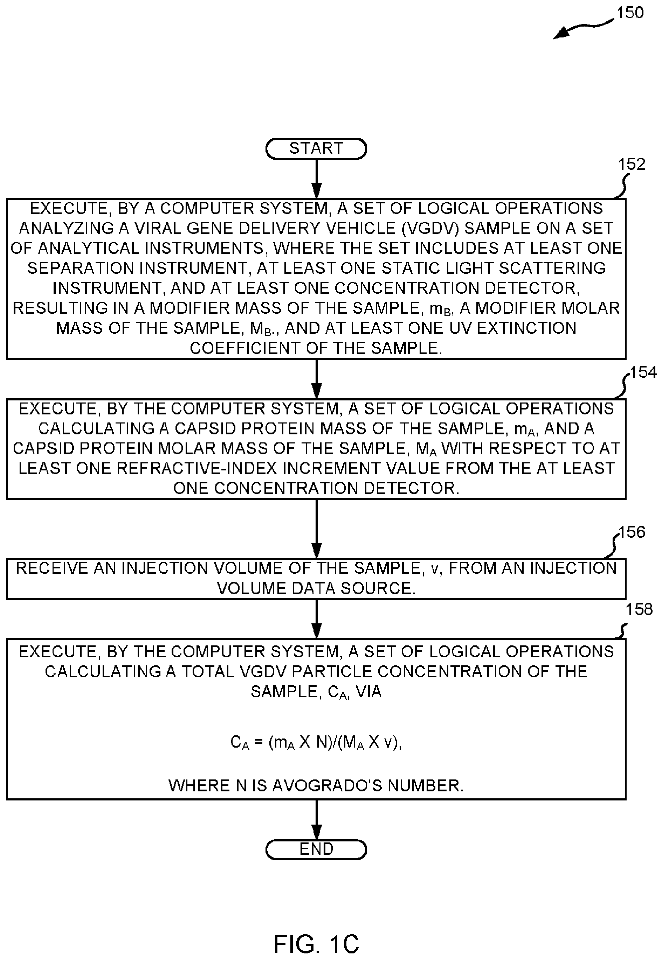

Referring to FIG. 1C, in an exemplary embodiment, the computer implemented method, the system, and the computer program product are configured to perform an operation 152 of executing, by a computer system, a set of logical operations analyzing a viral gene delivery vehicle (VGDV) sample on a set of analytical instruments, where the set includes at least one separation instrument, at least one static light scattering (SLS) instrument, and at least one concentration detector, resulting in a modifier mass of the sample, m_{B}, a modifier molar mass of the sample, M_{B}, and at least one UV extinction coefficient of the sample, an operation 154 of executing, by the computer system, a set of logical operations calculating a capsid protein mass of the sample, m_{A}, and a capsid protein molar mass of the sample, M_{A}, with respect to at least one refractive-index increment value from the at least one concentration detector, an operation 156 of receiving an injection volume of the sample, v, from an injection volume data source, and (4) an operation 158 of executing, by the computer system, a set of logical operations calculating a total VGDV particle concentration of the sample, C_{A}, via ${C}_{A} = \left({m}_{A} x N\right) / \left({M}_{A} x v\right) ,$ where N is Avogrado's number.

In an exemplary embodiment, the computer system is a standalone computer system, such as computer system 800 shown in FIG. 8, a network of distributed computers, where at least some of the computers are computer systems such as computer system 800 shown in FIG. 8, or a cloud computing node server, such as computer system 800 shown in FIG. 8. In an embodiment, the computer system is a computer system 800 as shown in FIG. 8, that executes a measuring attributes of a viral gene delivery vehicle (VGDV) sample via separation script or computer software application that carries out the operations of at least method 150. In an embodiment, the computer system is a computer system/server 812 as shown in FIG. 8, that executes a measuring attributes of a viral gene delivery vehicle (VGDV) sample via separation script or computer software application that carries out the operations of at least method 150. In an embodiment, the computer system is a processing unit 816 as shown in FIG. 8, that executes a measuring attributes of a viral gene delivery vehicle (VGDV) sample via separation script or computer software application that carries out the operations of at least method 150. In an embodiment, the computer system is a processor of the analytical instrument, that executes a measuring attributes of a viral gene delivery vehicle (VGDV) sample via separation script or computer software application that carries out the operations of at least method 150.

In an embodiment, the computer system is a computer system 800 as shown in FIG. 8, that executes a measuring attributes of a viral gene delivery vehicle (VGDV) sample via separation script or computer software application that carries out at least operations 152, 154, 156, and 158. In an embodiment, the computer system is a computer system/server 812 as shown in FIG. 8, that executes a measuring attributes of a viral gene delivery vehicle (VGDV) sample via separation script or computer software application that carries out at least operations 152, 154, 156, and 158. In an embodiment, the computer system is a processing unit 816 as shown in FIG. 8, that executes a measuring attributes of a viral gene delivery vehicle (VGDV) sample via separation script or computer software application that carries out at least operations 152, 154, 156, and 158.

### Total VGDV Particle Concentration with UV Detectors

In an exemplary embodiment, the computer implemented method, the system, and the computer program product include (1) executing, by a computer system, a set of logical operations analyzing a viral gene delivery vehicle (VGDV) sample on a set of analytical instruments, where the set includes at least one separation instrument, at least one static light scattering (SLS) instrument, and at least two concentration detectors, (2) executing, by the computer system, a set of logical operations calculating a mass fraction of a protein in the sample, X_{A}, with respect to an ultra-violet absorbance value, A^{λ1}, collected from the sample at a first wavelength, λ1, an ultra-violet absorbance value, A^{λ2}, collected from the sample at a second wavelength, λ2, an extinction coefficient of the protein, ε_{A}^{λ1}, at the first wavelength, λ1, an extinction coefficient of the protein, ε_{A}^{λ2}, at the second wavelength, λ2, an extinction coefficient of a modifier in the sample, ε_{B}^{λ1}, at the first wavelength, λ1, and an extinction coefficient of the modifier in the sample, ε_{B}^{λ2}, at the second wavelength, λ2, (3) executing, by the computer system, a set of logical operations calculating an extinction coefficient of the sample at the first wavelength, ε_{VGDV}^{λ1}, with respect to the mass fraction of the protein in the sample, X_{A}, the extinction coefficient of the protein at the first wavelength, ε_{A}^{λ1}, and the extinction coefficient of the modifier in the sample at the first wavelength, ε_{B}^{λ1}, (4) executing, by the computer system, a set of logical operations calculating an extinction coefficient of the sample at the second wavelength, ε_{VGDV}^{λ2}, with respect to the mass fraction of the protein in the sample, X_{A}, the extinction coefficient of the protein at the second wavelength, ε_{A}^{λ2}, and the extinction coefficient of the modifier in the sample at the second wavelength, ε_{B}^{λ2}, (5) executing, by the computer system, a set of logical operations calculating a refractive index increment of the sample, (dn/dc)_{VGDV}, with respect to the mass fraction of the protein in the sample, X_{A}, a refractive index coefficient of the protein (dn/dc)_{A}, and a refractive index coefficient of the modifier in the sample (dn/dc)_{B}, (6) executing, by the computer system, a set of logical operations calculating a total mass of the protein, m_{A}, and a total mass of the modifier, m_{B}, with respect to an ultra-violet absorbance value, A^{λ}, collected from the sample at a wavelength, λ, the mass fraction of the protein in the sample, X_{A}, an extinction coefficient of the protein, ε_{A}^{λ}, at the wavelength, λ, an extinction coefficient of a modifier in the sample, ε_{B}^{λ}, at the wavelength, λ, where the wavelength, λ, one of the first wavelength, λ1, and the second wavelength, λ2, and (7) executing, by the computer system, a set of logical operations calculating a total VGDV particle concentration of the sample, C_{A}, via ${C}_{A} = \left({m}_{A} x N\right) / \left({M}_{A} x v\right) ,$ where N is Avogrado's number, and where M_{A} is a capsid protein molar mass of the sample from a capsid protein molar mass data source. In an embodiment, the at least two concentration detectors include a first ultra-violet absorbance (UV) detector at the first wavelength, λ1, and a second ultra-violet absorbance (UV) detector at the second wavelength, λ2. In an embodiment, the at least two concentration detectors are a first ultra-violet absorbance (UV) detector at the first wavelength, λ1, and a second ultra-violet absorbance (UV) detector at the second wavelength, λ2.

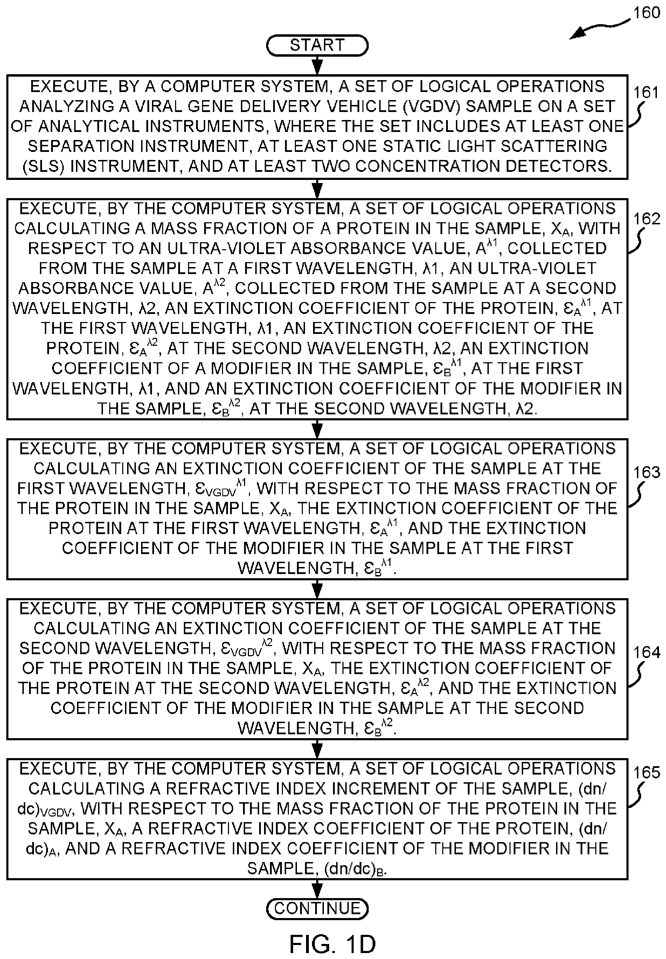

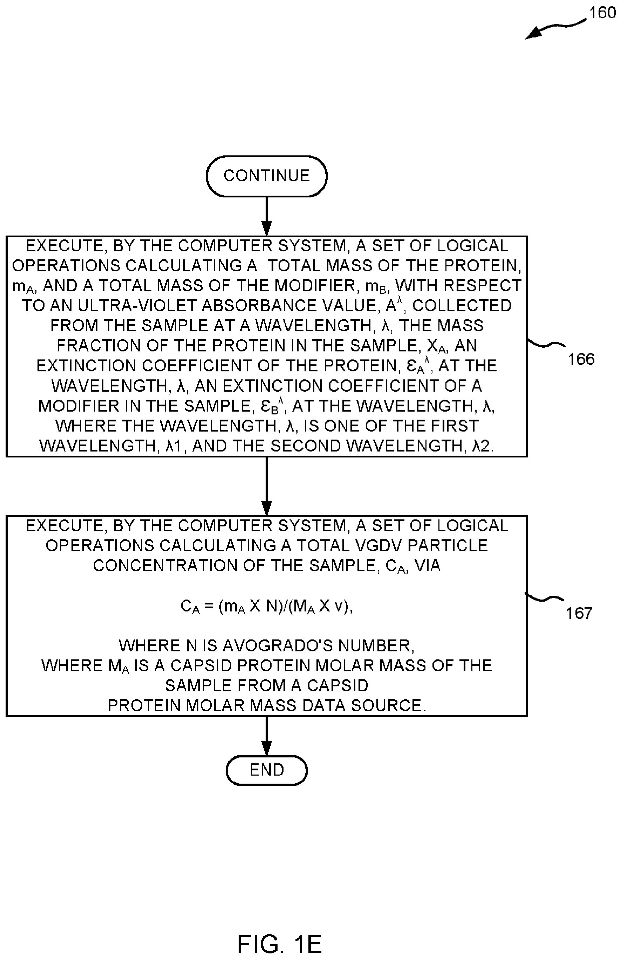

Referring to FIG. 1D and FIG. 1E, in an exemplary embodiment, the computer implemented method, the system, and the computer program product are configured to perform an operation 161 of executing, by a computer system, a set of logical operations analyzing a viral gene delivery vehicle (VGDV) sample on a set of analytical instruments, where the set includes at least one separation instrument, at least one static light scattering (SLS) instrument, and at least two concentration detectors, an operation 162 of executing, by the computer system, a set of logical operations calculating a mass fraction of a protein in the sample, X_{A}, with respect to an ultra-violet absorbance value, A^{λ1}, collected from the sample at a first wavelength, λ1, an ultra-violet absorbance value, A^{λ2}, collected from the sample at a second wavelength, λ2, an extinction coefficient of the protein, ε_{A}^{λ1}, at the first wavelength, λ1, an extinction coefficient of the protein, ε_{A}^{λ2}, at the second wavelength, λ2, an extinction coefficient of a modifier in the sample, ε_{B}^{λ1}, at the first wavelength, λ1, and an extinction coefficient of the modifier in the sample, ε_{B}^{λ2}, at the second wavelength, λ2, an operation 163 of executing, by the computer system, a set of logical operations calculating an extinction coefficient of the sample at the first wavelength, ε_{VGDV}^{λ1}, with respect to the mass fraction of the protein in the sample, X_{A}, the extinction coefficient of the protein at the first wavelength, ε_{A}^{λ1}, and the extinction coefficient of the modifier in the sample at the first wavelength, ε_{B}^{λ1}, an operation 164 of executing, by the computer system, a set of logical operations calculating an extinction coefficient of the sample at the second wavelength, ε_{VGDV}^{λ2}, with respect to the mass fraction of the protein in the sample, X_{A}, the extinction coefficient of the protein at the second wavelength, ε_{A}^{λ2}, and the extinction coefficient of the modifier in the sample at the second wavelength, ε_{B}^{λ2}, an operation 165 of executing, by the computer system, a set of logical operations calculating a refractive index increment of the sample, (dn/dc)_{VGDV}, with respect to the mass fraction of the protein in the sample, X_{A}, a refractive index coefficient of the protein (dn/dc)_{A}, and a refractive index coefficient of the modifier in the sample (dn/dc)_{B}, an operation 166 of executing, by the computer system, a set of logical operations calculating a total mass of the protein, m_{A}, and a total mass of the modifier, m_{B}, with respect to an ultra-violet absorbance value, A^{λ}, collected from the sample at a wavelength, λ, the mass fraction of the protein in the sample, X_{A}, an extinction coefficient of the protein, ε_{A}^{λ}, at the wavelength, λ, an extinction coefficient of a modifier in the sample, ε_{B}^{λ}, at the wavelength, λ, where the wavelength, λ, one of the first wavelength, λ1, and the second wavelength, λ2, and an operation 167 of executing, by the computer system, a set of logical operations calculating a total VGDV particle concentration of the sample, C_{A}, via ${C}_{A} = \left({m}_{A} x N\right) / \left({M}_{A} x v\right) ,$ where N is Avogrado's number, and where M_{A} is a capsid protein molar mass of the sample from a capsid protein molar mass data source.

In an exemplary embodiment, the computer system is a standalone computer system, such as computer system 800 shown in FIG. 8, a network of distributed computers, where at least some of the computers are computer systems such as computer system 800 shown in FIG. 8, or a cloud computing node server, such as computer system 800 shown in FIG. 8. In an embodiment, the computer system is a computer system 800 as shown in FIG. 8, that executes a measuring attributes of a viral gene delivery vehicle (VGDV) sample via separation script or computer software application that carries out the operations of at least method 160. In an embodiment, the computer system is a computer system/server 812 as shown in FIG. 8, that executes a measuring attributes of a viral gene delivery vehicle (VGDV) sample via separation script or computer software application that carries out the operations of at least method 160. In an embodiment, the computer system is a processing unit 816 as shown in FIG. 8, that executes a measuring attributes of a viral gene delivery vehicle (VGDV) sample via separation script or computer software application that carries out the operations of at least method 160. In an embodiment, the computer system is a processor of the analytical instrument, that executes a measuring attributes of a viral gene delivery vehicle (VGDV) sample via separation script or computer software application that carries out the operations of at least method 160.

In an embodiment, the computer system is a computer system 800 as shown in FIG. 8, that executes a measuring attributes of a viral gene delivery vehicle (VGDV) sample via separation script or computer software application that carries out at least operations 161, 162, 163, 164, 165, 166, and 167. In an embodiment, the computer system is a computer system/server 812 as shown in FIG. 8, that executes a measuring attributes of a viral gene delivery vehicle (VGDV) sample via separation script or computer software application that carries out at least operations 161, 162, 163, 164, 165, 166, and 167. In an embodiment, the computer system is a processing unit 816 as shown in FIG. 8, that executes a measuring attributes of a viral gene delivery vehicle (VGDV) sample via separation script or computer software application that carries out at least operations 161, 162, 163, 164, 165, 166, and 167.

In an embodiment, the calculating the mass fraction of the protein in the sample, X_{A}, includes calculating the mass fraction of the protein in the sample, X_{A}, via 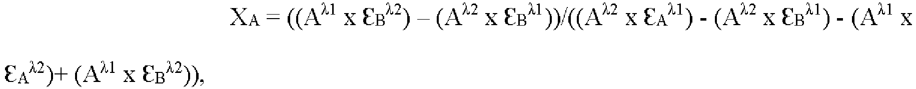 XA = ((Aλ1 x εBλ2) - (Aλ2 x εBλ1))/((Aλ2 x εAλ1) - (Aλ2 x εBλ1) - (Aλ1 x εAλ2)+ (Aλ1 x εBλ2)),
the calculating the extinction coefficient of the sample at the first wavelength, ε_{VGDV}^{λ1}, includes calculating the extinction coefficient of the sample at the first wavelength, ε_{VGDV}^{λ1}, via ${{ε}_{VGDV}}^{λ 1} = \left({X}_{A} x {{ε}_{A}}^{λ 1}\right) + \left(\left(1-{X}_{A}\right) x {{ε}_{B}}^{λ 1}\right) ,$
the calculating the extinction coefficient of the sample at the second wavelength, ε_{VGDV}^{λ2}, includes calculating the extinction coefficient of the sample at the second wavelength, ε_{VGDV}^{λ2}, via ${{ε}_{VGDV}}^{λ 2} = \left({X}_{A} x {{ε}_{A}}^{λ 2}\right) + \left(\left(1-{X}_{A}\right) x {{ε}_{B}}^{λ 2}\right) ,$
the calculating the refractive index increment of the sample, (dn/dc)_{VGDV}, includes calculating the refractive index increment of the sample, (dn/dc)_{VGDV}, via ${\left(dn/dc\right)}_{VGDV} = \left({X}_{A} x {\left(dn/dc\right)}_{A}\right) + \left(\left(1-{X}_{A}\right) x {\left(dn/dc\right)}_{B}\right) ,$
the calculating the total mass of the protein, m_{A}, includes calculating the total mass of the protein, m_{A}, via ${m}_{A} = \left({A}^{λ} x {X}_{A}\right) / \left(\left({X}_{A} x {{ε}_{A}}^{λ}\right)+\left(\left(1-{X}_{A}\right) x {{ε}_{B}}^{λ}\right)\right) ,$
and the calculating the total mass of the modifier, m_{B}, includes calculating the total mass of the modifier, m_{B}, via ${m}_{B} = \left({A}^{λ} x \left(1-{X}_{A}\right)\right) / \left(\left({X}_{A} x {{ε}_{A}}^{λ}\right)+\left(\left(1-{X}_{A}\right) x {{ε}_{B}}^{λ}\right)\right) .$

In an embodiment, calculating operation 162 of calculating the mass fraction of a protein in the sample, X_{A}, includes calculating the mass fraction of a protein in the sample, X_{A}, via 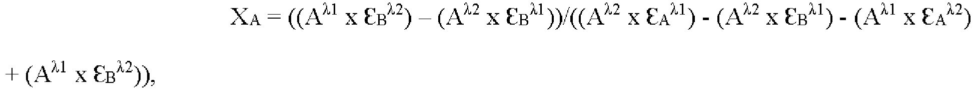 XA = ((Aλ1 x εBλ2) - (Aλ2 x εBλ1))/((Aλ2 x εAλ1) - (Aλ2 x εBλ1) - (Aλ1 x εAλ2) + (Aλ1 x εBλ2)),
calculating operation 163 of calculating the extinction coefficient of the sample at the first wavelength, ε_{VGDV}^{λ1}, includes calculating the extinction coefficient of the sample at the first wavelength, ε_{VGDV}^{λ1}, via ${{ε}_{VGDV}}^{λ 1} = \left({X}_{A}\times {{ε}_{A}}^{λ 1}\right) + \left(\left(1-{X}_{A}\right)\times {{ε}_{B}}^{λ 1}\right) ,$
calculating operation 164 of calculating the extinction coefficient of the sample at the second wavelength, ε_{VGDV}^{λ2}, includes calculating the extinction coefficient of the sample at the second wavelength, ε_{VGDV}^{λ2}, via ${{ε}_{VGDV}}^{λ 2} = \left({X}_{A}\times {{ε}_{A}}^{λ 2}\right) + \left(\left(1-{X}_{A}\right)\times {{ε}_{B}}^{λ 2}\right) ,$
calculating operation 165 of calculating the refractive index increment of the sample, (dn/dc)_{VGDV}, includes calculating the refractive index increment of the sample, (dn/dc)_{VGDV}, via ${\left(dn/dc\right)}_{VGDV} = \left({X}_{A}\times {\left(dn/dc\right)}_{A}\right) + \left(\left(1-{X}_{A}\right)\times {\left(dn/dc\right)}_{B}\right) ,$
calculating operation 166 of calculating the total mass of the protein, m_{A}, includes calculating the total mass of the protein, m_{A}, via ${m}_{A} = \left({A}^{λ}\times {X}_{A}\right) / \left(\left({X}_{A}\times {{ε}_{A}}^{λ}\right)+\left(\left(1-{X}_{A}\right)\times {{ε}_{B}}^{λ}\right)\right) ,$
and calculating operation 167 of calculating the total mass of the modifier, m_{B}, includes calculating the total mass of the modifier, m_{B}, via ${m}_{B} = \left({A}^{λ}\times \left(1-{X}_{A}\right)\right) / \left(\left({X}_{A}\times {{ε}_{A}}^{λ}\right)+\left(\left(1-{X}_{A}\right)\times {{ε}_{B}}^{λ}\right)\right) .$

In an embodiment, the first wavelength, λ1, is 260 nm, and the second wavelength, λ2, is 280 nm. In an embodiment, the modifier is a nucleic acid.

### Total VGDV Particle Concentration with UV Detector and dRI Detector

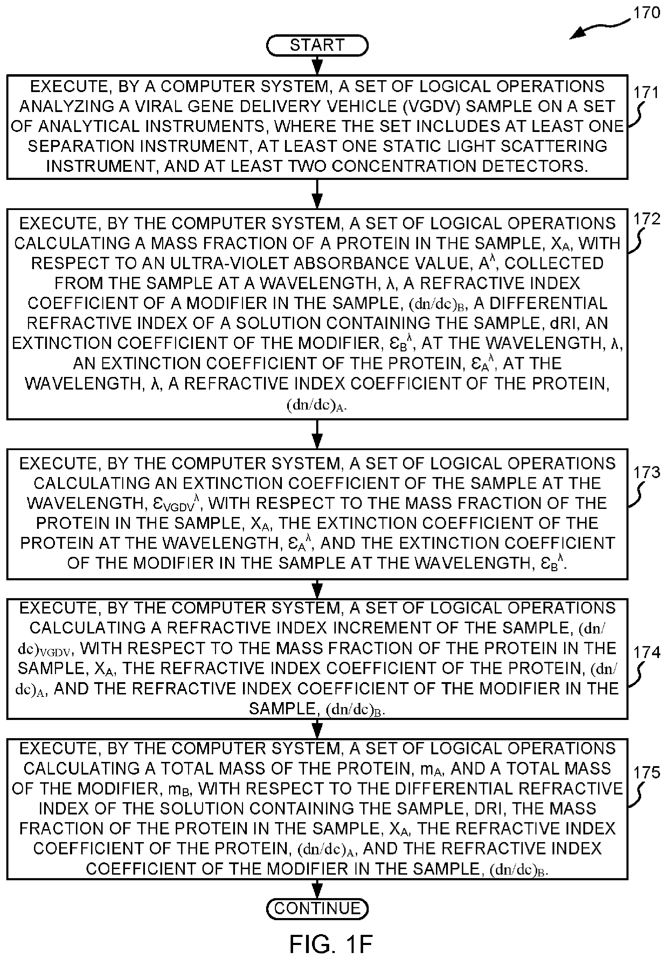

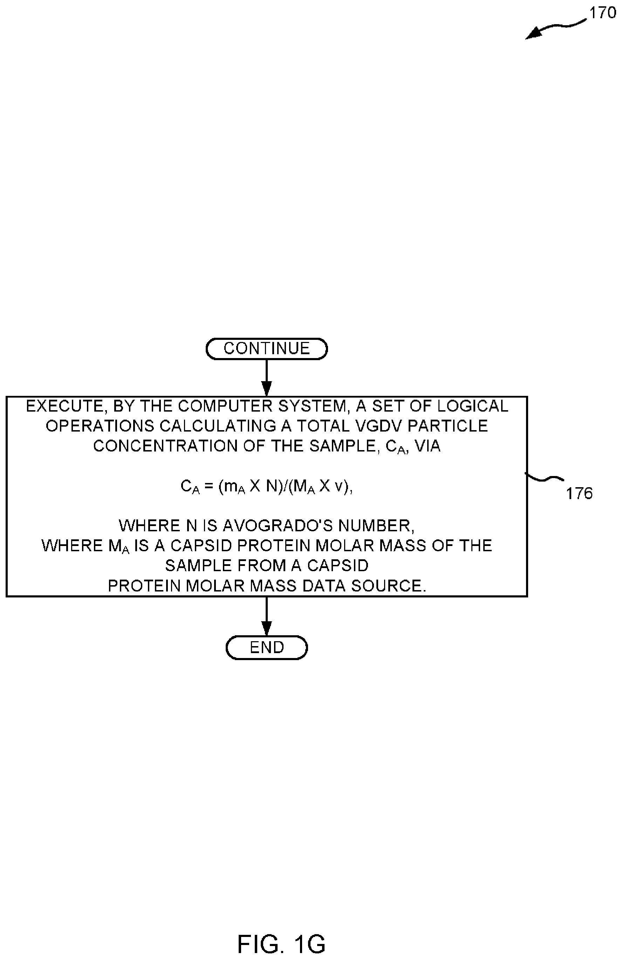

In an exemplary embodiment not falling under the scope of the present invention, the computer implemented method, the system, and the computer program product include (1) executing, by a computer system, a set of logical operations analyzing a viral gene delivery vehicle (VGDV) sample on a set of analytical instruments, where the set includes at least one separation instrument, at least one static light scattering (SLS) instrument, and at least two concentration detectors, (2) executing, by the computer system, a set of logical operations calculating a mass fraction of a protein in the sample, X_{A}, with respect to an ultra-violet absorbance value, A^{λ}, collected from the sample at a wavelength, λ, a refractive index coefficient of a modifier in the sample, (dn/dc)_{B}, a differential refractive index of a solution containing the sample, dRI, an extinction coefficient of the modifier, ε_{B}^{λ}, at the wavelength, λ, an extinction coefficient of the protein, ε_{A}^{λ}, at the wavelength, λ, a refractive index coefficient of the protein, (dn/dc)_{A}, (3) executing, by the computer system, a set of logical operations calculating an extinction coefficient of the sample at the wavelength, ε_{VGDV}^{λ}, with respect to the mass fraction of the protein in the sample, X_{A}, the extinction coefficient of the protein at the first wavelength, ε_{A}^{λ}, and the extinction coefficient of the modifier in the sample at the wavelength, ε_{B}^{λ}, (4) executing, by the computer system, a set of logical operations calculating a refractive index increment of the sample, (dn/dc)_{VGDV}, with respect to the mass fraction of the protein in the sample, X_{A}, the refractive index coefficient of the protein, (dn/dc)_{A}, and the refractive index coefficient of the modifier in the sample, (dn/dc)_{B}, (5) executing, by the computer system, a set of logical operations calculating a total mass of the protein, m_{A}, and a total mass of the modifier, m_{B}, with respect to the differential refractive index of the solution containing the sample, dRI, the mass fraction of the protein in the sample, X_{A}, the refractive index coefficient of the protein, (dn/dc)_{A}, and the refractive index coefficient of the modifier in the sample, (dn/dc)_{B}, and (6) executing, by the computer system, a set of logical operations calculating a total VGDV particle concentration of the sample, C_{A}, via ${C}_{A} = \left({m}_{A}\times N\right) / \left({M}_{A}\times v\right) ,$ where N is Avogrado's number, and where M_{A} is a capsid protein molar mass of the sample from a capsid protein molar mass data source. In an embodiment, the at least two concentration detectors include an ultra-violet absorbance (UV) detector at the wavelength, λ, and a differential refractive index (dRI) detector. In an embodiment, the at least two concentration detectors are a UV detector at the wavelength, λ, and a dRI detector.
Referring to FIG. 1F and 1G, in an exemplary embodiment, the computer implemented method, the system, and the computer program product are configured to perform an operation 171 of executing, by a computer system, a set of logical operations analyzing a viral gene delivery vehicle (VGDV) sample on a set of analytical instruments, where the set includes at least one separation instrument, at least one static light scattering (SLS) instrument, and at least two concentration detectors, an operation 172 of executing, by the computer system, a set of logical operations calculating a mass fraction of a protein in the sample, X_{A}, with respect to an ultra-violet absorbance value, A^{λ}, collected from the sample at a wavelength, λ, a refractive index coefficient of a modifier in the sample, (dn/dc)_{B}, a differential refractive index of a solution containing the sample, dRI, an extinction coefficient of the modifier, ε_{B}^{λ}, at the wavelength, λ, an extinction coefficient of the protein, ε_{A}^{λ}, at the wavelength, λ, a refractive index coefficient of the protein, (dn/dc)_{A}, an operation 173 of executing, by the computer system, a set of logical operations calculating an extinction coefficient of the sample at the wavelength, ε_{VGDV}^{λ}, with respect to the mass fraction of the protein in the sample, X_{A}, the extinction coefficient of the protein at the wavelength, ε_{A}^{λ}, and the extinction coefficient of the modifier in the sample at the wavelength, ε_{B}^{λ}, an operation 174 of executing, by the computer system, a set of logical operations calculating a refractive index increment of the sample, (dn/dc)_{VGDV}, with respect to the mass fraction of the protein in the sample, X_{A}, the refractive index coefficient of the protein, (dn/dc)_{A}, and the refractive index coefficient of the modifier in the sample, (dn/dc)_{B}, an operation 175 of executing, by the computer system, a set of logical operations calculating a total mass of the protein, m_{A}, and a total mass of the modifier, m_{B}, with respect to the differential refractive index of the solution containing the sample, dRI, the mass fraction of the protein in the sample, X_{A}, the refractive index coefficient of the protein, (dn/dc)_{A}, and the refractive index coefficient of the modifier in the sample, (dn/dc)_{B}, and an operation 176 of executing, by the computer system, a set of logical operations calculating a total VGDV particle concentration of the sample, C_{A}, via ${C}_{A} = \left({m}_{A}\times N\right) / \left({M}_{A}\times v\right) ,$ where N is Avogrado's number, and where M_{A} is a capsid protein molar mass of the sample from a capsid protein molar mass data source.

In an exemplary embodiment, the computer system is a standalone computer system, such as computer system 800 shown in FIG. 8, a network of distributed computers, where at least some of the computers are computer systems such as computer system 800 shown in FIG. 8, or a cloud computing node server, such as computer system 800 shown in FIG. 8. In an embodiment, the computer system is a computer system 800 as shown in FIG. 8, that executes a measuring attributes of a viral gene delivery vehicle (VGDV) sample via separation script or computer software application that carries out the operations of at least method 170. In an embodiment, the computer system is a computer system/server 812 as shown in FIG. 8, that executes a measuring attributes of a viral gene delivery vehicle (VGDV) sample via separation script or computer software application that carries out the operations of at least method 170. In an embodiment, the computer system is a processing unit 816 as shown in FIG. 8, that executes a measuring attributes of a viral gene delivery vehicle (VGDV) sample via separation script or computer software application that carries out the operations of at least method 170. In an embodiment, the computer system is a processor of the analytical instrument, that executes a measuring attributes of a viral gene delivery vehicle (VGDV) sample via separation script or computer software application that carries out the operations of at least method 170.

In an embodiment, the computer system is a computer system 800 as shown in FIG. 8, that executes a measuring attributes of a viral gene delivery vehicle (VGDV) sample via separation script or computer software application that carries out at least operations 171, 172, 173, 174, 175, and 176. In an embodiment, the computer system is a computer system/server 812 as shown in FIG. 8, that executes a measuring attributes of a viral gene delivery vehicle (VGDV) sample via separation script or computer software application that carries out at least operations 171, 172, 173, 174, 175, and 176. In an embodiment, the computer system is a processing unit 816 as shown in FIG. 8, that executes a measuring attributes of a viral gene delivery vehicle (VGDV) sample via separation script or computer software application that carries out at least operations 171, 172, 173, 174, 175, and 176.

In an embodiment not falling under the scope of the present invention, the calculating the mass fraction of the protein in the sample, X_{A}, includes calculating the mass fraction of the protein in the sample, X_{A}, via 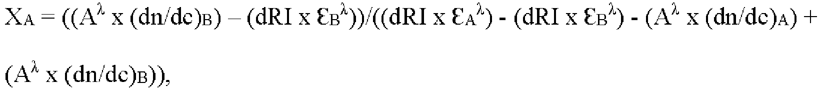 XA = ((Aλ x (dn/dc)B) - (dRI x εBλ))/((dRI x εAλ) - (dRI x εBλ) - (Aλ x (dn/dc)A) + (Aλ x (dn/dc)B)),
the calculating the extinction coefficient of the sample at the wavelength, ε_{VGDV}^{λ}, includes calculating the extinction coefficient of the sample at the wavelength, ε_{VGDV}^{λ}, via ${{ε}_{VGDV}}^{λ} = \left({X}_{A}\times {{ε}_{A}}^{λ}\right) + \left(\left(1-{X}_{A}\right)\times {{ε}_{B}}^{λ}\right) ,$
the calculating the refractive index increment of the sample, (dn/dc)_{VGDV}, includes calculating the refractive index increment of the sample, (dn/dc)_{VGDV}, via ${\left(dn/dc\right)}_{VGDV} = \left({X}_{A}\times {\left(dn/dc\right)}_{A}\right) + \left(\left(1-{X}_{A}\right)\times {\left(dn/dc\right)}_{B}\right) ,$
the calculating the total mass of the protein, m_{A}, includes calculating the total mass of the protein, m_{A}, via ${m}_{A} = \left(dRI\times {X}_{A}\right) / \left(\left({X}_{A}\times {\left(dn/dc\right)}_{A}\right)+\left(\left(1-{X}_{A}\right)\times {\left(dn/dc\right)}_{B}\right)\right) ,$
and the calculating the total mass of the modifier, m_{B}, includes calculating the total mass of the modifier, m_{B}, via ${m}_{B} = \left(dRI x \left(1-{X}_{A}\right)\right) / \left(\left({X}_{A} x {\left(dn/dc\right)}_{A}\right)+\left(\left(1-{X}_{A}\right) x {\left(dn/dc\right)}_{B}\right)\right) .$

In an embodiment, the wavelength, λ, is one of 260 nm and 280 nm. In an embodiment, the modifier is a nucleic acid.

### Total VGDV Particle Concentration with UV Detector and FLD

In an embodiment, the computer implemented method, the system, and the computer program product include (1) executing, by a computer system, a set of logical operations analyzing a viral gene delivery vehicle (VGDV) sample on a set of analytical instruments, where the set includes at least one separation instrument, at least one static
light scattering (SLS) instrument, and at least two concentration detectors, (2) executing, by the computer system, a set of logical operations calculating a mass fraction of a protein in the sample, X_{A}, with respect to an area under a peak of fluorescence emission data collected from the sample, FLD, at an excitation wavelength, an ultra-violet absorbance value collected from the sample, A^{λ}, at an ultraviolet wavelength, λ, an extinction coefficient of the protein, ε_{A}^{λ}, at the ultraviolet wavelength, λ, a proportionality constant relating a concentration of the protein with a fluorescence intensity relating to FLD, ε_{FLD,A}, an extinction coefficient of a modifier in the sample, ε_{B}^{λ}, at the ultraviolet wavelength, λ, and a proportionality constant relating a concentration of the modifier with a fluorescence intensity relating to FLD, ε_{FLD,B}, (3) executing, by the computer system, a set of logical operations calculating an extinction coefficient of the sample at the ultraviolet wavelength, ε_{VGDV}^{λ}, with respect to the mass fraction of the protein in the sample, X_{A}, the extinction coefficient of the protein at the ultraviolet wavelength, ε_{A}^{λ}, and the extinction coefficient of the modifier in the sample at the ultraviolet wavelength, ε_{B}^{λ}, (4) executing, by the computer system, a set of logical operations calculating an extinction coefficient of the sample at the excitation wavelength, ε_{FLD,VGDV}, with respect to the mass fraction of the protein in the sample, X_{A}, the proportionality constant relating the concentration of the protein with the fluorescence intensity relating to FLD, ε_{FLD,A}, and the proportionality constant relating the concentration of the modifier with the fluorescence intensity relating to FLD, ε_{FLD,B}, (5) executing, by the computer system, a set of logical operations calculating a refractive index increment of the sample, (dn/dc)_{VGDV}, with respect to the mass fraction of the protein in the sample, X_{A}, a refractive index coefficient of the protein (dn/dc)_{A}, and a refractive index coefficient of the modifier in the sample (dn/dc)_{B}, (6) executing, by the computer system, a set of logical operations calculating a total mass of the protein, m_{A}, and a total mass of the modifier, m_{B}, with respect to the area under the peak of the fluorescence emission data collected from the sample, FLD, at the excitation wavelength, the mass fraction of the protein in the sample, X_{A}, the proportionality constant relating the concentration of the protein with the fluorescence intensity relating to FLD, ε_{FLD,A}, and the proportionality constant relating the concentration of the modifier with the fluorescence intensity relating to FLD, ε_{FLD,B}, and (7) executing, by the computer system, a set of logical operations calculating a total VGDV particle concentration of the sample, C_{A}, via ${C}_{A} = \left({m}_{A} x N\right) / \left({M}_{A} x v\right) ,$ where N is Avogrado's number, and where M_{A} is a capsid protein molar mass of the sample from a capsid protein molar mass data source. In an embodiment, the at least two concentration detectors include an ultra-violet absorbance (UV) detector at the ultraviolet wavelength, λ, and a fluorescence detector (FLD). In an embodiment, the at least two concentration detectors are a UV detector at the ultraviolet wavelength, λ, and a FLD.

In an embodiment, the computer implemented method, the system, and the computer program product are configured to perform an operation of executing, by a computer system, a set of logical operations analyzing a viral gene delivery vehicle (VGDV) sample on a set of analytical instruments, where the set includes at least one separation instrument, at least one static light scattering (SLS) instrument, and at least two concentration detectors, an operation of executing, by the computer system, a set of logical operations calculating a mass fraction of a protein in the sample, X_{A}, with respect to an area under a peak of fluorescence emission data collected from the sample, FLD, at an excitation wavelength, an ultra-violet absorbance value collected from the sample, A^{λ}, at an ultraviolet wavelength, λ, an extinction coefficient of the protein, ε_{A}^{λ}, at the ultraviolet wavelength, λ, a proportionality constant relating a concentration of the protein with a fluorescence intensity relating to FLD, ε_{FLD,A}, an extinction coefficient of a modifier in the sample, ε_{B}^{λ}, at the ultraviolet wavelength, λ, and a proportionality constant relating a concentration of the modifier with a fluorescence intensity relating to FLD, ε_{FLD,B}, an operation of executing, by the computer system, a set of logical operations calculating an extinction coefficient of the sample at the ultraviolet wavelength, ε_{VGDV}^{λ}, with respect to the mass fraction of the protein in the sample, X_{A}, the extinction coefficient of the protein at the ultraviolet wavelength, ε_{A}^{λ}, and the extinction coefficient of the modifier in the sample at the ultraviolet wavelength, ε_{B}^{λ}, an operation of executing, by the computer system, a set of logical operations calculating an extinction coefficient of the sample at the excitation wavelength, ε_{FLD,VGDV}, with respect to the mass fraction of the protein in the sample, X_{A}, the proportionality constant relating the concentration of the protein with the fluorescence intensity relating to FLD, ε_{FLD,A}, and the proportionality constant relating the concentration of the modifier with the fluorescence intensity relating to FLD, ε_{FLD,B}, an operation of executing, by the computer system, a set of logical operations calculating a refractive index increment of the sample, (dn/dc)_{VGDV}, with respect to the mass fraction of the protein in the sample, X_{A}, a refractive index coefficient of the protein (dn/dc)_{A}, and a refractive index coefficient of the modifier in the sample (dn/dc)_{B}, an operation of executing, by the computer system, a set of logical operations calculating a total mass of the protein, m_{A}, and a total mass of the modifier, m_{B}, with respect to the area under the peak of the fluorescence emission data collected from the sample, FLD, at the excitation wavelength, the mass fraction of the protein in the sample, X_{A}, the proportionality constant relating the concentration of the protein with the fluorescence intensity relating to FLD, ε_{FLD,A}, and the proportionality constant relating the concentration of the modifier with the fluorescence intensity relating to FLD, ε_{FLD,B}, and an operation of executing, by the computer system, a set of logical operations calculating a total VGDV particle concentration of the sample, C_{A}, via ${C}_{A} = \left({m}_{A} x N\right) / \left({M}_{A} x v\right) ,$ where N is Avogrado's number, and where M_{A} is a capsid protein molar mass of the sample from a capsid protein molar mass data source.

In an embodiment, the calculating the mass fraction of the protein in the sample, X_{A}, includes calculating the mass fraction of the protein in the sample, X_{A}, via 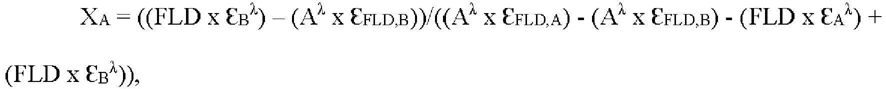 XA = ((FLD x εBλ) - (Aλ x εFLD,B))/((Aλ x εFLD,A) - (Aλ x εFLD,B) - (FLD x εAλ) + (FLD x εBλ)),
the calculating the extinction coefficient of the sample at the ultraviolet wavelength, ε_{VGDV}^{λ}, includes calculating the extinction coefficient of the sample at the ultraviolet wavelength, ε_{VGDV}^{λ}, via ${{ε}_{VGDV}}^{λ} = \left({X}_{A} x {{ε}_{A}}^{λ}\right) + \left(\left(1-{X}_{A}\right) x {{ε}_{B}}^{λ}\right) ,$
the calculating the extinction coefficient of the sample at the excitation wavelength, ε_{FLD,VGDV}, includes calculating the extinction coefficient of the sample at the excitation wavelength, ε_{FLD,VGDV}, via ${ε}_{FLD , VGDV} = \left({X}_{A} x {ε}_{FLD , A}\right) + \left(\left(1-{X}_{A}\right) x {ε}_{FLD , B}\right) ,$ and
the calculating the refractive index increment of the sample, (dn/dc)_{VGDV}, includes calculating the refractive index increment of the sample, (dn/dc)_{VGDV} via ${\left(dn/dc\right)}_{VGDV} = \left({X}_{A} x {\left(dn/dc\right)}_{A}\right) + \left(\left(1-{X}_{A}\right) x {\left(dn/dc\right)}_{B}\right) ,$
the calculating the total mass of the protein, m_{A}, includes calculating the total mass of the protein, m_{A}, via ${m}_{A} = \left(FLD x {X}_{A}\right) / \left(\left({X}_{A} x {ε}_{FLD , A}\right)+\left(\left(1-{X}_{A}\right) x {ε}_{FLD , B}\right)\right) ,$
and the calculating the total mass of the modifier, m_{B}, includes calculating the total mass of the modifier, m_{B}, via ${m}_{B} = \left(FLD x \left(1-{X}_{A}\right)\right) / \left(\left({X}_{A} x {ε}_{FLD , A}\right)+\left(\left(1-{X}_{A}\right) x {ε}_{FLD , B}\right)\right) .$

In an embodiment, the UV wavelength, λ, is one of 260 nm and 280 nm. In an embodiment, the modifier is a nucleic acid.

### Total VGDV Particle Concentration with dRI and FLD

In an embodiment, the computer implemented method, the system, and the computer program product include (1) executing, by a computer system, a set of logical operations analyzing a viral gene delivery vehicle (VGDV) sample on a set of analytical instruments, where the set includes at least one separation instrument, at least one static light scattering (SLS) instrument, and at least two concentration detectors (2) executing, by the computer system, a set of logical operations calculating a mass fraction of a protein in the sample, X_{A}, with respect to an area under a peak of fluorescence emission data collected from the sample, FLD, at an excitation wavelength, a differential refractive index of a solution containing the sample, dRI, a refractive index coefficient of the protein, (dn/dc)_{A}, a refractive index coefficient of a modifier, (dn/dc)_{B}, in the sample, a proportionality constant relating a concentration of the protein with a fluorescence intensity relating to FLD, ε_{FLD,A}, a proportionality constant relating a concentration of the modifier with a fluorescence intensity relating to FLD, ε_{FLD,B}, (3) executing, by the computer system, a set of logical operations calculating a refractive index increment of the sample, (dn/dc)_{VGDV}, with respect to mass fraction of the protein in the sample, X_{A}, the refractive index coefficient of the protein, (dn/dc)_{A}, the, and the refractive index coefficient of the modifier in the sample, (dn/dc)_{B}, (4) executing, by the computer system, a set of logical operations calculating an extinction coefficient of the sample at the excitation wavelength, ε_{FLD,VGDV}, with respect to the mass fraction of the protein in the sample, X_{A}, the proportionality constant relating the concentration of the protein with the fluorescence intensity relating to FLD, ε_{FLD,A}, and the proportionality constant relating the concentration of the modifier with the fluorescence intensity relating to FLD, ε_{FLD,B}, and (5) executing, by the computer system, a set of logical operations calculating a total VGDV particle concentration of the sample, C_{A}, via ${C}_{A} = \left({m}_{A} x N\right) / \left({M}_{A} x v\right) ,$ where N is Avogrado's number, where m_{A} is a total mass of the protein, and where M_{A} is a capsid protein molar mass of the sample from a capsid protein molar mass data source. In an embodiment, the at least two concentration detectors include a differential refractive index (dRI) detector and a fluorescence detector (FLD). In an embodiment, the at least two concentration detectors are a dRI detector and a FLD.

In an embodiment, the computer implemented method, the system, and the computer program product are configured to perform an operation of executing, by a computer system, a set of logical operations analyzing a viral gene delivery vehicle (VGDV) sample on a set of analytical instruments, where the set includes at least one separation instrument, at least one static light scattering (SLS) instrument, and at least two concentration detectors, and operation of executing, by the computer system, a set of logical operations calculating a mass fraction of a protein in the sample, X_{A}, with respect to an area under a peak of fluorescence emission data collected from the sample, FLD, at an excitation wavelength, a differential refractive index of a solution containing the sample, dRI, a refractive index coefficient of the protein, (dn/dc)_{A}, a refractive index coefficient of a modifier, (dn/dc)_{B}, in the sample, a proportionality constant relating a concentration of the protein with a fluorescence intensity relating to FLD, ε_{FLD,A}, a proportionality constant relating a concentration of the modifier with a fluorescence intensity relating to FLD, ε_{FLD,B}, an operation of executing, by the computer system, a set of logical operations calculating a refractive index increment of the sample, (dn/dc)_{VGDV}, with respect to mass fraction of the protein in the sample, X_{A}, the refractive index coefficient of the protein, (dn/dc)_{A}, the, and the refractive index coefficient of the modifier in the sample, (dn/dc)_{B}, an operation of executing, by the computer system, a set of logical operations calculating an extinction coefficient of the sample at the excitation wavelength, ε_{FLD,VGDV}, with respect to the mass fraction of the protein in the sample, X_{A}, the proportionality constant relating the concentration of the protein with the fluorescence intensity relating to FLD, ε_{FLD,A}, and the proportionality constant relating the concentration of the modifier with the fluorescence intensity relating to FLD, ε_{FLD,B}, and an operation of executing, by the computer system, a set of logical operations calculating a total VGDV particle concentration of the sample, C_{A}, via ${C}_{A} = \left({m}_{A} x N\right) / \left({M}_{A} x v\right) ,$ where N is Avogrado's number, where m_{A} is a total mass of the protein, and where M_{A} is a capsid protein molar mass of the sample from a capsid protein molar mass data source.

In an embodiment, the calculating the mass fraction of the protein in the sample, X_{A}, includes calculating the mass fraction of the protein in the sample, X_{A}, via $\begin{matrix}{X}_{A} = \left(\left(FLD\times {\left(dn/dc\right)}_{B}\right)-\left(dRI\times {ε}_{FLD , B}\right)\right) / \\ \left(\left(dRI\times {ε}_{FLD , A}\right)-\left(dRI\times {ε}_{FLD , B}\right)-\left(FLD\times {\left(dn/dc\right)}_{A}\right)+\left(FLD\times {\left(dn/dc\right)}_{B}\right)\right) ,\end{matrix}$
the calculating the refractive index increment of the sample, (dn/dc)_{VGDV}, includes calculating the refractive index increment of the sample, (dn/dc)_{VGDV}, via ${\left(dn/dc\right)}_{VGDV} = \left({X}_{A}\times {\left(dn/dc\right)}_{A}\right) + \left(\left(1-{X}_{A}\right)\times {\left(dn/dc\right)}_{B}\right) ,$
and the calculating the extinction coefficient of the sample at the excitation wavelength, ε_{FLD,VGDV}, includes calculating the extinction coefficient of the sample at the excitation wavelength, ε_{FLD,VGDV}, via ${ε}_{FLD , VGDV} = \left({X}_{A}\times {ε}_{FLD , A}\right) + \left(\left(1-{X}_{A}\right)\times {ε}_{FLD , B}\right) .$

### Example

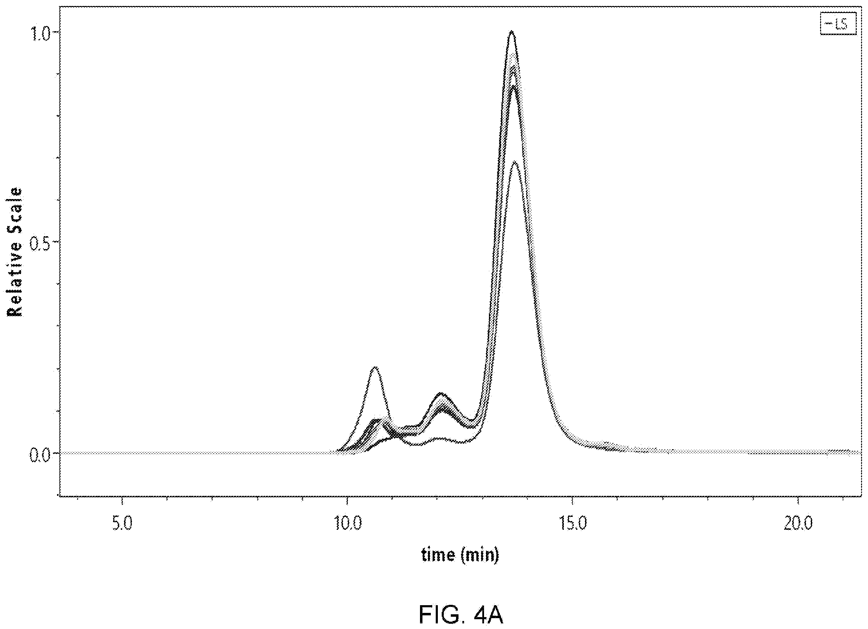

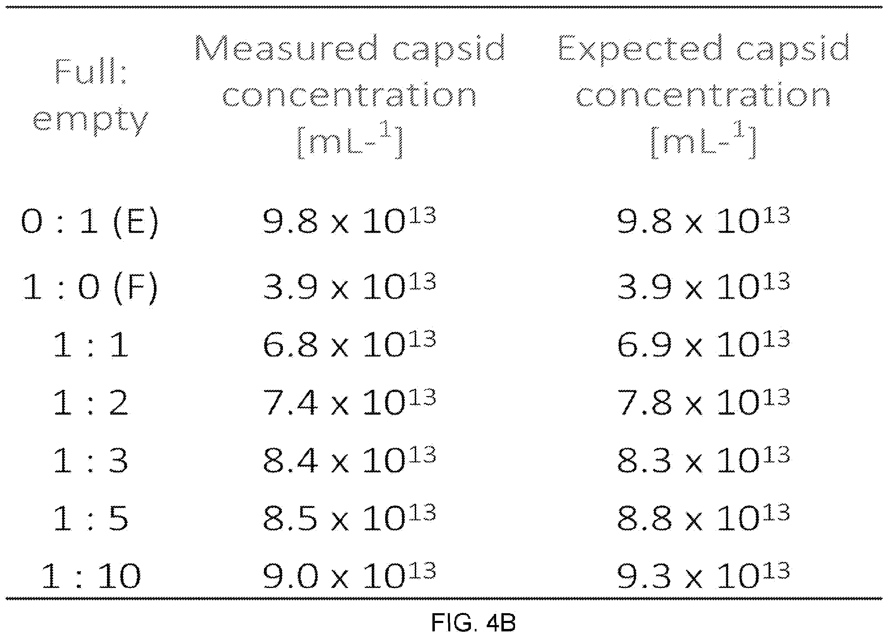

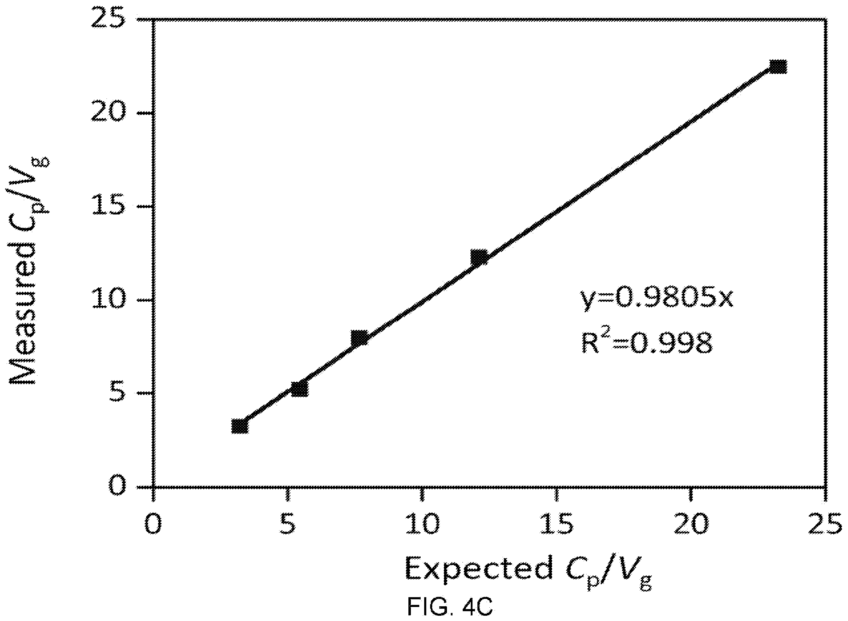

For example, FIG. 4A, FIG. 4B, and FIG. 4C depict the consistency with which the method could calculate particle concentration, the total VGDV particle concentration of a sample (C_{A}), where "Full : Empty" reflects a mixing ratio of a first sample to a second sample. FIG. 4C notably demonstrates that the method could calculate capsid content of a sample (Cₚ / V_{g}), (C_{A} / C_{FULL})), well. FIG. 4A shows representative data (chromatograms) collected by the SLS instrument at 90 degrees. FIG. 4B demonstrates that the method could calculate the total VGDV concentration of a sample.

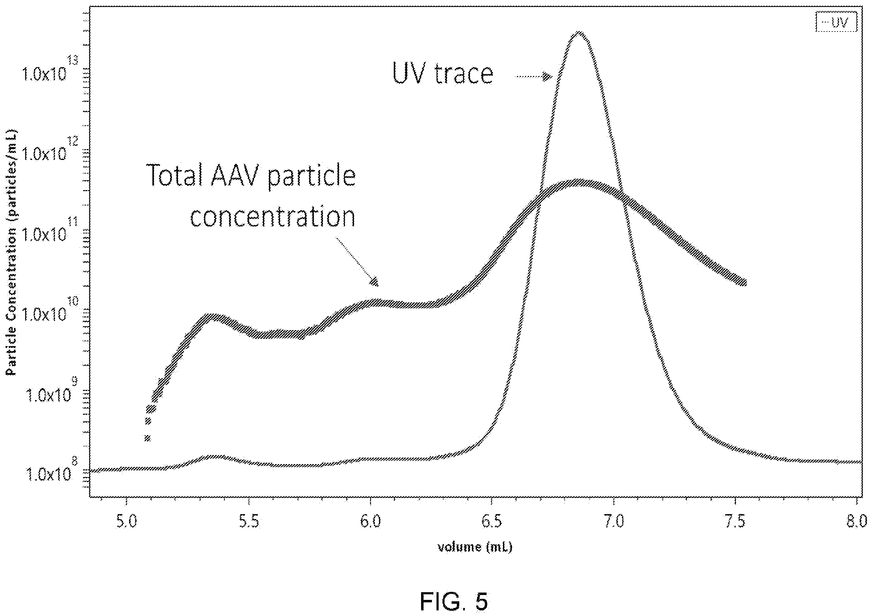

FIG. 5. depicts the ability of the method to quantify aggregation content of an AAV sample. FIG. 5 shows total AAV particle concentration overlaid with a UV trace of the AAV sample. The peaks eluted before the main peak (6.5 mL-7.5 mL) are AAV aggregates. Using the method, the degree of aggregation is determined by calculating the particle concentration of all of the eluting data slices (chromatogram data slices).

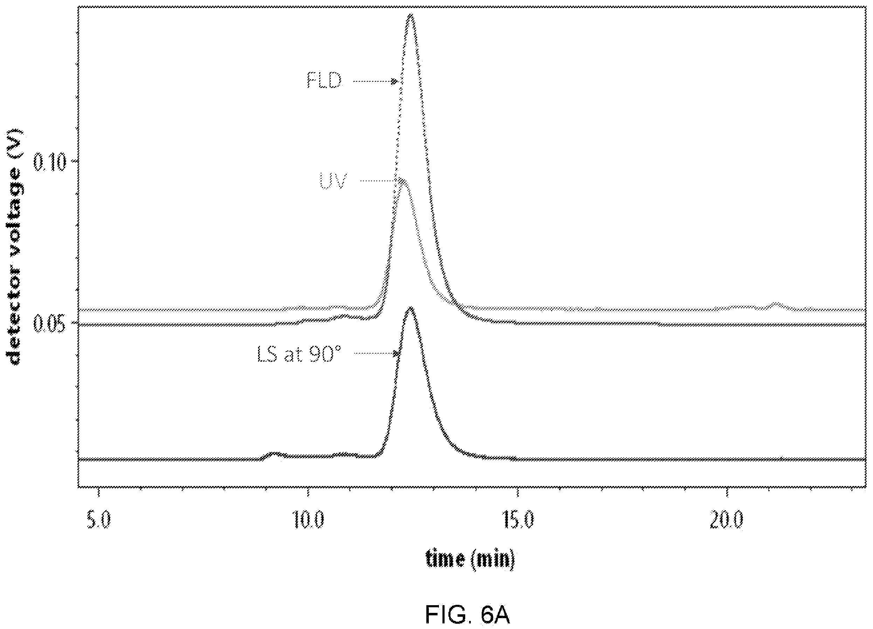

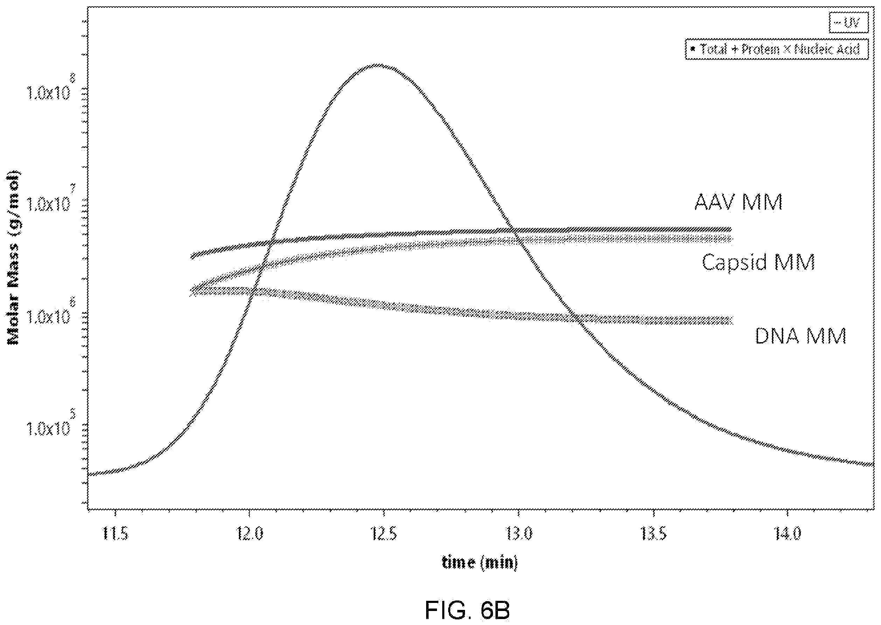

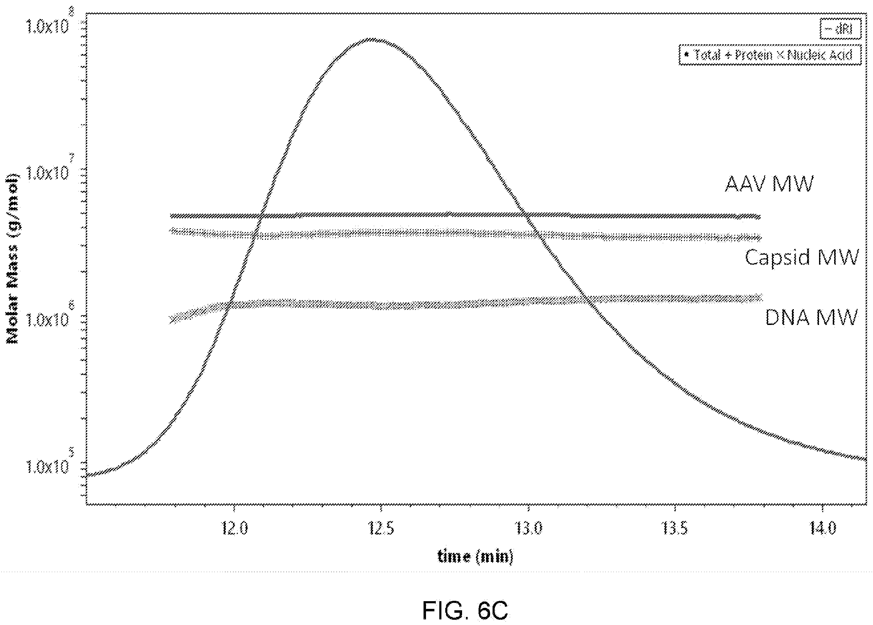

FIG. 6A shows that a fluorescence detector (FLD) may also be used as one of the concentration detectors. FIG. 6A shows representative data (chromatograms) collected by the SLS instrument at 90 degrees, a UV detector, and a FLD. FIG. 6B depicts the molar mass (MM) results/molecular weight (MW) results of analyzing an AAV sample via the method using UV and FLD as the concentration detectors, which are consistent with expected molar mass values/expected molecular weight results. FIG. 6C depicts molecular weight (MW) results/molar mass (MM) results of analyzing an AAV sample via the method using dRI and FLD as the concentration detector, which are consistent with expected molecular weight values/expected molar mass values.

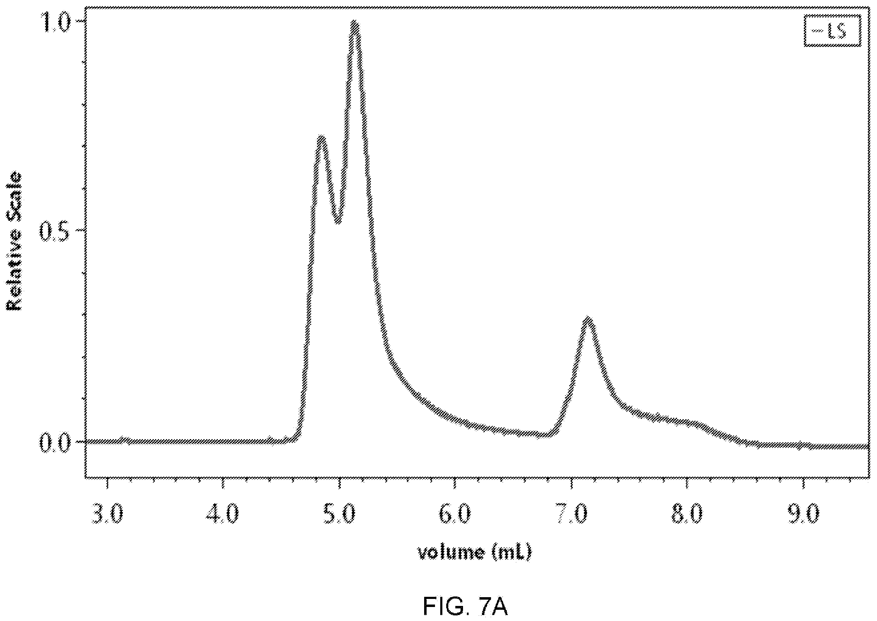

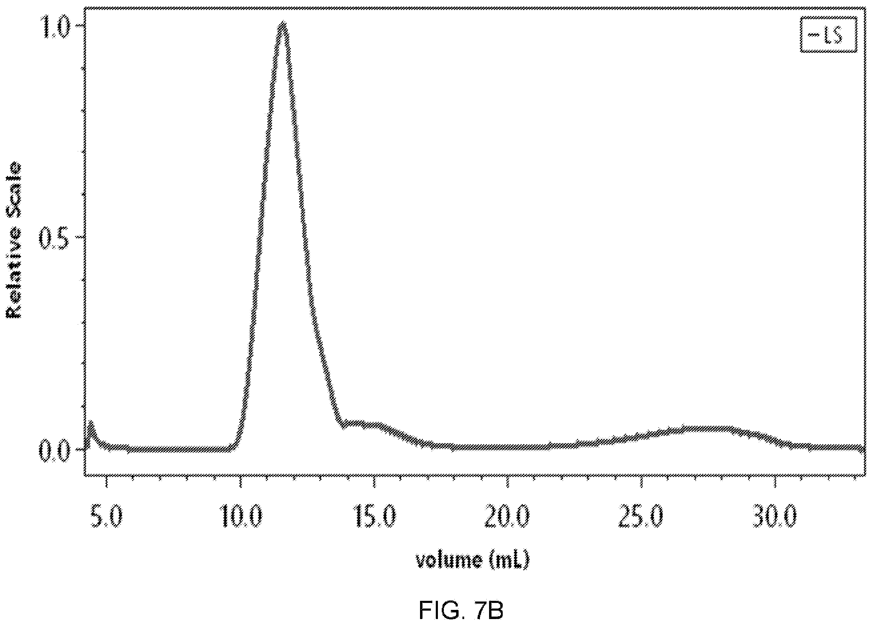

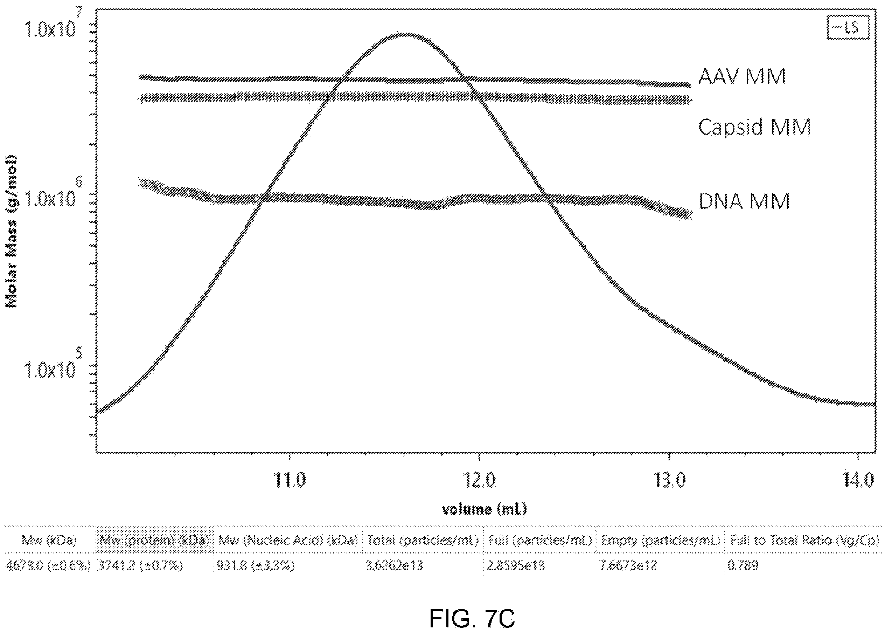

FIG. 7A shows data collected via the method using ion-exchange chromatography (IEX) as the separation instrument. FIG. 7A shows representative data (chromatograms) collected by the SLS instrument at 90 degrees. FIG. 7B shows data collected via the method using field flow fractionation (FFF) as the separation instrument. FIG. 7B shows representative data (chromatograms) collected by the SLS instrument at 90 degrees. FIG. 7C depicts the molar mass (MM) results of analyzing an AAV sample via the method using UV and dRI as the concentration detectors and FFF as the separation system, which are consistent with expected molar mass values/expected molecular weight values.

### Computer System

In an exemplary embodiment, the computer system is a computer system 500 as shown in FIG. 5. Computer system 500 is only one example of a computer system and is not intended to suggest any limitation as to the scope of use or functionality of embodiments of the present invention. Regardless, computer system 500 is capable of being implemented to perform and/or performing any of the functionality/operations of the present invention.

Computer system 500 includes a computer system/server 512, which is operational with numerous other general purpose or special purpose computing system environments or configurations. Examples of well-known computing systems, environments, and/or configurations that may be suitable for use with computer system/server 512 include, but are not limited to, personal computer systems, server computer systems, thin clients, thick clients, handheld or laptop devices, multiprocessor systems, microprocessor-based systems, set top boxes, programmable consumer electronics, network PCs, minicomputer systems, mainframe computer systems, and distributed cloud computing environments that include any of the above systems or devices.

Computer system/server 512 may be described in the general context of computer system-executable instructions, such as program modules, being executed by a computer system. Generally, program modules may include routines, programs, objects, components, logic, and/or data structures that perform particular tasks or implement particular abstract data types. Computer system/server 512 may be practiced in distributed cloud computing environments where tasks are performed by remote processing devices that are linked through a communications network. In a distributed cloud computing environment, program modules may be located in both local and remote computer system storage media including memory storage devices.

As shown in FIG. 5, computer system/server 512 in computer system 500 is shown in the form of a general-purpose computing device. The components of computer system/server 512 may include, but are not limited to, one or more processors or processing units 516, a system memory 528, and a bus 518 that couples various system components including system memory 528 to processor 516.

Bus 518 represents one or more of any of several types of bus structures, including a memory bus or memory controller, a peripheral bus, an accelerated graphics port, and a processor or local bus using any of a variety of bus architectures. By way of example, and not limitation, such architectures include Industry Standard Architecture (ISA) bus, Micro Channel Architecture (MCA) bus, Enhanced ISA (EISA) bus, Video Electronics Standards Association (VESA) local bus, and Peripheral Component Interconnects (PCI) bus.

Computer system/server 512 typically includes a variety of computer system readable media. Such media may be any available media that is accessible by computer system/server 512, and includes both volatile and non-volatile media, removable and non-removable media.

System memory 528 can include computer system readable media in the form of volatile memory, such as random access memory (RAM) 530 and/or cache memory 532. Computer system/server 512 may further include other removable/non-removable, volatile/non-volatile computer system storage media. By way of example only, storage system 534 can be provided for reading from and writing to a non-removable, non-volatile magnetic media (not shown and typically called a "hard drive"). Although not shown, a magnetic disk drive for reading from and writing to a removable, non-volatile magnetic disk (e.g., a "floppy disk"), and an optical disk drive for reading from or writing to a removable, non-volatile optical disk such as a CD-ROM, DVD-ROM or other optical media can be provided. In such instances, each can be connected to bus 518 by one or more data media interfaces. As will be further depicted and described below, memory 528 may include at least one program product having a set (e.g., at least one) of program modules that are configured to carry out the functions/operations of embodiments of the invention.

Program/utility 540, having a set (at least one) of program modules 542, may be stored in memory 528 by way of example, and not limitation. Exemplary program modules 542 may include an operating system, one or more application programs, other program modules, and program data. Each of the operating system, one or more application programs, other program modules, and program data or some combination thereof, may include an implementation of a networking environment. Program modules 542 generally carry out the functions and/or methodologies of embodiments of the present invention.

Computer system/server 512 may also communicate with one or more external devices 514 such as a keyboard, a pointing device, a display 524, one or more devices that enable a user to interact with computer system/server 512, and/or any devices (e.g., network card, modem, etc.) that enable computer system/server 512 to communicate with one or more other computing devices. Such communication can occur via Input/Output (I/O) interfaces 522. Still yet, computer system/server 512 can communicate with one or more networks such as a local area network (LAN), a general wide area network (WAN), and/or a public network (e.g., the Internet) via network adapter 520. As depicted, network adapter 520 communicates with the other components of computer system/server 512 via bus 518. It should be understood that although not shown, other hardware and/or software components could be used in conjunction with computer system/server 512. Examples, include, but are not limited to: microcode, device drivers, redundant processing units, external disk drive arrays, RAID systems, tape drives, and data archival storage systems.

### Computer Program Product

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The descriptions of the various embodiments of the present disclosure have been presented for purposes of illustration, but are not intended to be exhaustive or limited to the embodiments disclosed. Many modifications and variations will be apparent to those of ordinary skill in the art without departing from the scope of the described embodiments. The terminology used herein was chosen to explain the principles of the embodiments, the practical application or technical improvement over technologies found in the marketplace, or to enable others of ordinary skill in the art to understand the embodiments disclosed herein.

## Claims

1. A computer implemented method comprising:
executing, by a computer system, a set of logical operations analyzing a viral gene delivery vehicle (VGDV) sample on a set of analytical instruments,
wherein the set comprises at least one separation instrument, at least one static light scattering instrument, and at least two concentration detectors,
resulting in a capsid protein mass of the sample, m_{A}, a modifier mass of the sample, m_{B}, and a modifier molar mass of the sample, M_{B} (110);
receiving a capsid protein molar mass of the sample, M_{A}, from a capsid protein molar mass data source (112);
receiving an injection volume of the sample, v, from an injection volume data source (114); and
executing, by the computer system, a set of logical operations calculating a total VGDV particle concentration of the sample, C_{A}, via ${C}_{A} = \left({m}_{A}\times N\right) / \left({M}_{A}\times v\right) ,$ wherein N is Avogrado's number (116).

2. The method of claim 1 further comprising:
executing, by the computer system, a set of logical operations analyzing the sample on the set, resulting in the capsid protein molar mass of the sample, M_{A}; and
storing the capsid protein molar mass of the sample, M_{A}, in the capsid protein molar mass data source.

3. The method of claim 1 wherein the analyzing comprises analyzing the sample on the set via an analysis technique,
wherein the analysis technique is one of viral vector analysis, protein conjugate analysis, and copolymer composition analysis.

4. The method of claim 1 wherein the at least two concentration detectors comprise a first ultra-violet absorbance detector at a first wavelength, λ1, and a second ultra-violet absorbance detector at a second wavelength, λ2.

5. The method of claim 1 wherein the at least two concentration detectors comprise an ultra-violet absorbance detector at a wavelength, λ, and a differential refractive index detector.

6. The method of claim 1 wherein the at least two concentration detectors comprise an ultra-violet absorbance detector at a wavelength, λ, and a fluorescence detector.

7. The method of claim 1 further comprising:
receiving, by the computer system, a molar mass of a full modifier inside a full VGDV sample, M_{Full}, from a full modifier molar mass data source;
executing, by the computer system, a set of logical operations calculating a full VGDV concentration of the full VGDV sample, C_{Full}, via ${C}_{Full} = \left({m}_{B}\times N\right) / \left({M}_{Full}\times v\right) ;$ and
executing, by the computer system, a set of logical operations calculating an empty VGDV concentration of the full VGDV sample, C_{Empty}, via ${C}_{Empty} = {C}_{A} - {C}_{Full} .$

8. The method of claim 7 further comprising
executing, by the computer system, a set of logical operations analyzing the full VGDV sample on the set,
resulting in the molar mass of the full modifier inside the full VGDV sample, M_{Full}; and
storing the molar mass of the full modifier inside the full VGDV sample, M_{Full}, in the full modifier molar mass data source.

9. The method of claim 1 further comprising:
executing, by the computer system, a set of logical operations analyzing an entire VGDV signal region of the sample on the set and analyzing an aggregate peak region of the sample on the set,
resulting in a VGDV entire peak protein mass of the sample, m_{A}, ₑₙₜ, corresponding to the entire VGDV signal region of the sample, a VGDV entire peak modifier mass of the sample, m_{B}, ₑₙₜ, corresponding to the entire VGDV signal region of the sample, a VGDV entire peak protein molar mass of the sample, M_{A}, ₑₙₜ, corresponding to the entire VGDV signal region of the sample, a VGDV entire peak modifier molar mass of the sample, M_{B}, ₑₙₜ, corresponding to the entire VGDV signal region of the sample, a VGDV aggregate peak protein mass of the sample, m_{A, agg}, corresponding to the aggregate peak region of the sample, a VGDV aggregate peak modifier mass of the sample, m_{B, agg}, corresponding to the aggregate peak region of the sample, a VGDV aggregate peak protein molar mass of the sample, M_{A, agg}, corresponding to the aggregate peak region of the sample, a VGDV aggregate peak modifier molar mass of the sample, M_{B, agg}, corresponding to the aggregate peak region of the sample; and
executing, by the computer system, a set of logical operations calculating a total VGDV entire peak particle concentration of the sample, C_{A, ent}, corresponding to the entire VGDV signal region of the sample, via ${C}_{A , ent} = \left({m}_{A , ent}\times N\right) / \left({M}_{A , ent}\times v\right) ;$ and
executing, by the computer system, a set of logical operations calculating a total VGDV aggregate peak particle concentration of the sample, C_{A, agg}, corresponding to the aggregate peak region of the sample, via ${C}_{A , agg} = \left({m}_{A , agg}\times N\right) / \left({M}_{A , agg}\times v\right) .$

10. The method of claim 9 further comprising:
executing, by the computer system, a set of logical operations analyzing an entire VGDV signal region of a full viral gene delivery vehicle sample on the set and analyzing an aggregate peak region of the full sample on the set,
resulting in a VGDV entire peak molar mass of a full modifier inside the full sample, M_{Full}, ₑₙₜ, corresponding to the entire VGDV signal region of the full sample, and a VGDV aggregate peak molar mass of the full modifier inside the full sample, M_{Full}, _{agg}, corresponding to the aggregate peak region of the full sample;
executing, by the computer system, a set of logical operations calculating a VGDV entire peak full VGDV concentration of the full sample, C_{Full}, ₑₙₜ, corresponding to the entire VGDV signal region of the VGDV sample, via ${C}_{Full , ent} = \left({m}_{B , ent}\times N\right) / \left({M}_{Full , end}\times v\right) ;$
executing, by the computer system, a set of logical operations calculating a VGDV aggregate peak full VGDV concentration of the full sample, C_{Full}, _{agg}, corresponding to the aggregate peak region of the full sample, via ${C}_{Full , agg} = \left({m}_{B , agg}\times N\right) / \left({M}_{Full , agg}\times v\right) ;$
executing, by the computer system, a set of logical operations calculating a VGDV entire peak empty VGDV concentration of the full sample, C_{Empty, ent}, corresponding to the entire VGDV signal region of the full sample, via ${C}_{Empty , ent} = {C}_{A , ent} - {C}_{Full , ent} ;$ and
executing, by the computer system, a set of logical operations calculating a VGDV aggregate peak empty VGDV concentration of the full sample, C_{Empty, agg}, corresponding to the aggregate peak region of the full sample, via ${C}_{Empty , agg} = {C}_{A , agg} - {C}_{Full , agg} .$

11. A computer implemented method comprising:
executing, by a computer system, a set of logical operations analyzing a viral gene delivery vehicle (VGDV) sample on a set of analytical instruments,
wherein the set comprises at least one separation instrument, at least one static light scattering instrument, and at least one concentration detector,
resulting in a modifier mass of the sample, m_{B}, a modifier molar mass of the sample, M_{B}, and at least one UV extinction coefficient of the sample (152);
executing, by the computer system, a set of logical operations calculating a capsid protein mass of the sample, m_{A}, and a capsid protein molar mass of the sample, M_{A}, with respect to at least one refractive-index increment value from the at least one concentration detector (154);
receiving an injection volume of the sample, v, from an injection volume data source (156); and
executing, by the computer system, a set of logical operations calculating a total VGDV particle concentration of the sample, C_{A}, via ${C}_{A} = \left({m}_{A}\times N\right) / \left({M}_{A}\times v\right) ,$ wherein N is Avogrado's number (158).

12. A computer implemented method comprising:
executing, by a computer system, a set of logical operations analyzing a viral gene delivery vehicle (VGDV) sample on a set of analytical instruments,
wherein the set comprises at least one separation instrument, at least one static light scattering instrument, and at least two concentration detectors (161);
executing, by the computer system, a set of logical operations calculating a mass fraction of a protein in the sample, X_{A}, with respect to an ultra-violet absorbance value, A^{λ1}, collected from the sample at a first wavelength, λ1, an ultra-violet absorbance value, A^{λ2}, collected from the sample at a second wavelength, λ2, an extinction coefficient of the protein, ε_{A}^{λ1}, at the first wavelength, λ1, an extinction coefficient of the protein, ε_{A}^{λ2}, at the second wavelength, λ2, an extinction coefficient of a modifier in the sample, ε_{B}^{λ1}, at the first wavelength, λ1, and an extinction coefficient of the modifier in the sample, ε_{B}^{λ2}, at the second wavelength, λ2 (162);
executing, by the computer system, a set of logical operations calculating an extinction coefficient of the sample at the first wavelength, ε_{VGDV}^{λ1}, with respect to the mass fraction of the protein in the sample, X_{A}, the extinction coefficient of the protein at the first wavelength, ε_{A}^{λ1}, and the extinction coefficient of the modifier in the sample at the first wavelength, ε_{B}^{λ1} (163);
executing, by the computer system, a set of logical operations calculating an extinction coefficient of the sample at the second wavelength, ε_{VGDV}^{λ2}, with respect to the mass fraction of the protein in the sample, X_{A}, the extinction coefficient of the protein at the second wavelength, ε_{A}^{λ2}, and the extinction coefficient of the modifier in the sample at the second wavelength, ε_{B}^{λ2}(164);
executing, by the computer system, a set of logical operations calculating a refractive index increment of the sample, (dn/dc)_{VGDV}, with respect to the mass fraction of the protein in the sample, X_{A}, a refractive index coefficient of the protein, (dn/dc)_{A}, and a refractive index coefficient of the modifier in the sample, (dn/dc)_{B} (165);
executing, by the computer system, a set of logical operations calculating a total mass of the protein, m_{A}, and a total mass of the modifier, m_{B}, with respect to an ultra-violet absorbance value, A^{λ}, collected from the sample at a wavelength, λ, the mass fraction of the protein in the sample, X_{A}, an extinction coefficient of the protein, ε_{A}^{λ}, at the wavelength, λ, an extinction coefficient of a modifier in the sample, ε_{B}^{λ}, at the wavelength, λ, where the wavelength, λ, is one of the first wavelength, λ1, and the second wavelength, λ₂ (166); and
executing, by the computer system, a set of logical operations calculating a total VGDV particle concentration of the sample, C_{A}, via ${C}_{A} = \left({m}_{A}\times N\right) / \left({M}_{A}\times v\right) ,$
wherein N is Avogrado's number,
wherein M_{A} is a capsid protein molar mass of the sample from a capsid protein molar mass data source (167).

13. The method of claim 12
wherein the calculating the mass fraction of the protein in the sample, X_{A}, comprises calculating the mass fraction of the protein in the sample, X_{A}, via ${X}_{A} = \left(\left({A}^{λ 1}\times {{ε}_{B}}^{λ 2}\right)-\left({A}^{λ 2}\times {{ε}_{B}}^{λ 1}\right)\right) / \left(\left({A}^{λ 2}\times {{ε}_{A}}^{λ 1}\right)-\left({A}^{λ 2}\times {{ε}_{B}}^{λ 1}\right)-\left({A}^{λ 1}\times {{ε}_{A}}^{λ 2}\right)+\left({A}^{λ 1}\times {{ε}_{B}}^{λ 2}\right)\right) ,$
wherein the calculating the extinction coefficient of the sample at the first wavelength, ε_{VGDV}^{λ1}, comprises calculating the extinction coefficient of the sample at the first wavelength, ε_{VGDV}^{λ1}, via ${{ε}_{VGDV}}^{λ 1} = \left({X}_{A}\times {{ε}_{A}}^{λ 1}\right) + \left(\left(1-{X}_{A}\right)\times {{ε}_{B}}^{λ 1}\right) ,$
wherein the calculating the extinction coefficient of the sample at the second wavelength, ε_{VGDV}^{λ2}, comprises calculating the extinction coefficient of the sample at the second wavelength, ε_{VGDV}^{λ2}, via ${{ε}_{VGDV}}^{λ 2} = \left({X}_{A}\times {{ε}_{A}}^{λ 2}\right) + \left(\left(1-{X}_{A}\right)\times {{ε}_{B}}^{λ 2}\right) ,$
wherein the calculating the refractive index increment of the sample, (dn/dc)_{VGDV}, comprises calculating the refractive index increment of the sample, (dn/dc)_{VGDV}, via ${\left(dn/dc\right)}_{VGDV} = \left({X}_{A}\times {\left(dn/dc\right)}_{A}\right) + \left(\left(1-{X}_{A}\right)\times {\left(dn/dc\right)}_{B}\right) ,$
wherein the calculating the total mass of the protein, m_{A}, comprises calculating the total mass of the protein, m_{A}, via ${m}_{A} = \left({A}^{λ}\times {X}_{A}\right) / \left(\left({X}_{A}\times {{ε}_{A}}^{λ}\right)+\left(\left(1-{X}_{A}\right)\times {{ε}_{B}}^{λ}\right)\right) ,$
wherein the calculating the total mass of the modifier, m_{B}, comprises calculating the total mass of the modifier, m_{B}, via ${m}_{B} = \left({A}^{λ}\times \left(1-{X}_{A}\right)\right) / \left(\left({X}_{A}\times {{ε}_{A}}^{λ}\right)+\left(\left(1-{X}_{A}\right)\times {{ε}_{B}}^{λ}\right)\right) .$

## Patentansprüche

1. Computerimplementiertes Verfahren, umfassend:
Ausführen, durch ein Computersystem, eines Satzes von logischen Operationen, die eine Probe eines Virusgenabgabevehikels (VGDV) auf einem Satz von Analyseinstrumenten analysieren,
wobei der Satz mindestens ein Trenninstrument, mindestens ein statisches Lichtstreuinstrument und mindestens zwei Konzentrationsdetektoren umfasst,
was eine Kapsidproteinmasse der Probe, m_{A}, eine Modifikatormasse der Probe, m_{B}, und eine Modifikator-Molmasse der Probe, M_{B} (110), ergibt;
Empfangen einer Kapsidprotein-Molmasse der Probe, M_{A}, von einer Kapsidprotein-Molmassen-Datenquelle (112);
Empfangen eines Injektionsvolumens der Probe, v, von einer Injektionsvolumen-Datenquelle (114), und
Ausführen, durch das Computersystem, eines Satzes von logischen Operationen, die eine absolute VGDV-Partikelkonzentration der Probe, C_{A}, BERECHNEN, mittels ${C}_{A} = \left({m}_{A} x N\right) / \left({M}_{A} x v\right) ,$ wobei N die Avogadro-Zahl ist (116).

2. Verfahren nach Anspruch 1, ferner umfassend:
Ausführen, durch das Computersystem, eines Satzes von logischen Operationen, die die Probe auf dem Satz analysieren, was die Kapsidprotein-Molmasse der Probe, M_{A}, ergibt; und
Speichern der Kapsidprotein-Molmasse der Probe, M_{A}, in der Kapsidprotein-Molmassen-Datenquelle.

3. Verfahren nach Anspruch 1, wobei das Analysieren das Analysieren der Probe auf dem Satz mittels einer Analysetechnik umfasst,
wobei die Analysetechnik eines von viraler Vektoranalyse, Proteinkonjugatanalyse und Copolymerzusammensetzungsanalyse ist.

4. Verfahren nach Anspruch 1, wobei die mindestens zwei Konzentrationsdetektoren einen ersten Ultraviolett-Absorptionsdetektor bei einer ersten Wellenlänge, λ1, und einen zweiten Ultraviolett-Absorptionsdetektor bei einer zweiten Wellenlänge, λ2, umfassen.

5. Verfahren nach Anspruch 1, wobei die mindestens zwei Konzentrationsdetektoren einen Ultraviolett-Absorptionsdetektor bei einer Wellenlänge, λ, und einen differentiellen Brechungsindex-Detektor umfassen.

6. Verfahren nach Anspruch 1, wobei die mindestens zwei Konzentrationsdetektoren einen Ultraviolett-Absorptionsdetektor bei einer Wellenlänge, λ, und einen Fluoreszenzdetektor umfassen.

7. Verfahren nach Anspruch 1, ferner umfassend:
Empfangen, durch das Computersystem, einer Molmasse eines vollen Modifikators innerhalb einer vollen VGDV-Probe, M_{Voll}, aus einer Voller-Modifikator-Molmassen-Datenquelle;
Ausführen, durch das Computersystem, eines Satzes von logischen Operationen, die eine Volles-VGDV-Konzentration der vollen VGDV-Probe, C_{Voll} berechnen, mittels ${C}_{Voll} = \left({m}_{B} x N\right) / \left({M}_{Voll} x V\right) ;$ und
Ausführen, durch das Computersystem, eines Satzes von logischen Operationen, die eine Leeres-VGDV-Konzentration der vollen VGDV-Probe, C_{Leer}, berechnen, mittels ${C}_{Leer} = {C}_{A} - {C}_{Voll} .$

8. Verfahren nach Anspruch 7, ferner umfassend
Ausführen, durch das Computersystem, eines Satzes von logischen Operationen, die die volle VGDV-Probe auf dem Satz analysieren,
was die Molmasse des vollen Modifikators innerhalb der vollen VGDV-Probe, M_{Voll}, ergibt; und
Speichern der Molmasse des vollen Modifikators innerhalb der vollen VGDV-Probe, M_{Voll}, in der Voller-Modifikator-Molmassen-Datenquelle.

9. Verfahren nach Anspruch 1, ferner umfassend:
Ausführen, durch das Computersystem, eines Satzes von logischen Operationen, die eine gesamte VGDV-Signalregion der Probe auf dem Satz analysieren und eine Aggregat-Peak-Region der Probe auf dem Satz analysieren,
was eine VGDV-Gesamt-Peak-Proteinmasse der Probe, m_{A}, _{ges (bzw. Gesamt)}, die der gesamten VGDV-Signalregion der Probe entspricht, eine VGDV-Gesamt-Peak-Modifikatormasse der Probe, m_{B}, _{ges (bzw. Gesamt)}, die der gesamten VGDV-Signalregion der Probe entspricht, eine VGDV-Gesamt-Peak-Proteinmolmasse der Probe,M_{A}, _{ges (bzw. Gesamt)}, die der gesamten VGDV-Signalregion der Probe entspricht, eine VGDV-Gesamt-Peak-Modifikator-Molmasse der Probe, M_{B}, _{ges (bzw. Gesamt)}, die der gesamten VGDV-Signalregion der Probe entspricht, eine VGDV-Aggregat-Peak-Proteinmasse der Probe, m_{A}, _{Agg (bzw. Aggregat)}, die der Aggregat-Peak-Region der Probe entspricht, eine VGDV-Aggregat-Peak-Modifikatormasse der Probe, m_{B}, _{Agg (bzw. Aggregat)}, die der Aggregat-Peak-Region der Probe entspricht, eine VGDV-Aggregat-Peak-Proteinmolmasse der Probe,M_{A}, _{Agg (bzw. Aggregat)}, die der Aggregat-Peak-Region der Probe entspricht, eine VGDV-Aggregat-Peak-Modifikator-Molmasse der Probe,M_{B}, _{Agg (bzw. Aggregat)}, die der Aggregat-Peak-Region der Probe entspricht, ergibt; und
Ausführen, durch das Computersystem, eines Satzes von logischen Operationen, die eine absolute VGDV-Gesamt-Peak-Partikelkonzentration der Probe, C_{A}, _{ges (bzw. Gesamt)} berechnen, die der gesamten VGDV-Signalregion der Probe entspricht, mittels ${C}_{A , ges \left(bzw. Gesamt\right)} = \left({m}_{A , ges \left(bzw. Gesamt\right)} x N\right) / \left({M}_{A , ges \left(gzw. Gesamt\right)} x v\right) ;$ und
Ausführen, durch das Computersystem, eines Satzes von logischen Operationen, die eine absolute VGDV-Aggregat-Peak-Partikelkonzentration der Probe, C_{A}, _{Agg (bzw. Aggregat)}, berechnen, die der Aggregat-Peak-Region der Probe entspricht, mittels ${C}_{A , Agg \left(bzw. Aggregat\right)} = \left({m}_{A , Agg \left(bzw. Aggregat\right)} x N\right) / \left({M}_{A , Agg \left(bzw. Aggregat\right)} x v\right) .$

10. Verfahren nach Anspruch 9, ferner umfassend:
Ausführen, durch das Computersystem, eines Satzes von logischen Operationen, die eine gesamte VGDV-Signalregion einer vollen Probe eines Virusgenabgabevehikels auf dem Satz analysieren und eine Aggregat-Peak-Region der vollen Probe auf dem Satz analysieren,
was eine VGDV-Gesamt-Peak-Molmasse eines vollen Modifikators innerhalb der vollen Probe, M_{Voll}, _{ges (bzw. Gesamt)}, die der gesamten VGDV-Signalregion der vollen Probe entspricht, und eine VGDV-Aggregat-Peak-Molmasse des vollen Modifikators innerhalb der vollen Probe, M_{Voll}, _{Agg (bzw. Aggregat)}, die der Aggregat-Peak-Region der vollen Probe entspricht, ergibt;
Ausführen, durch das Computersystem, eines Satzes von logischen Operationen, die eine VGDV-Gesamt-Peak-Volles-VGDV-Konzentration der vollen Probe, C_{Voll}, _{ges (bzw. Gesamt)}, berechnen, die der gesamten VGDV-Signalregion der VGDV-Probe entspricht, mittels ${C}_{Voll , ges \left(bzw. Gesamt\right)} = \left({m}_{B , ges \left(bzw. Gesamt\right)} x N\right) / \left({M}_{Voll , ges \left(bzw. Gesamt\right)} x v\right) ;$
Ausführen, durch das Computersystem, eines Satzes von logischen Operationen, die eine VGDV-Aggregat-Peak-Volles-VGDV-Konzentration der vollen Probe, C_{Voll}, _{Agg (bzw. Aggregat)}, berechnen, die der Aggregat-Peak-Region der vollen Probe entspricht, mittels ${C}_{Voll , Agg \left(bzw. Aggregat\right)} = \left({m}_{B , Agg \left(bzw. Aggregat\right)} x N\right) / \left({M}_{Voll , Agg \left(bzw. Aggregat\right)} x v\right)$
Ausführen, durch das Computersystem, eines Satzes von logischen Operationen, die eine VGDV-Gesamt-Peak-Leeres-VGDV-Konzentration der vollen Probe, C_{Leer}, _{ges (bzw. Gesamt)} berechnen, die der gesamten VGDV-Signalregion der vollen Probe entspricht, mittels ${C}_{Leer , ges \left(bzw. Gesamt\right)} = {C}_{A , ges \left(bzw. Gesamt\right)} - {C}_{Voll , ges \left(bzw. Gesamt\right)} , und$
Ausführen, durch das Computersystem, eines Satzes von logischen Operationen, die eine VGDV-Aggregat-Peak-Leeres-VGDV-Konzentration der vollen Probe, C_{Leer}, _{Agg (bzw. Aggregat)} berechnen, die der Aggregat-Peak-Region der vollen Probe entspricht, mittels ${C}_{Leer , Agg \left(bzw. Aggregat\right)} = {C}_{A , Agg \left(bzw. Aggregat\right)} - {C}_{Voll , Agg \left(bzw. Aggregat\right)} .$

11. Computerimplementiertes Verfahren, umfassend:
Ausführen, durch ein Computersystem, eines Satzes von logischen Operationen, die eine Probe eines Virusgenabgabevehikels (VGDV) auf einem Satz von Analyseinstrumenten analysieren,
wobei der Satz mindestens ein Trenninstrument, mindestens ein statisches Lichtstreuinstrument und mindestens einen Konzentrationsdetektor umfasst,
was eine Modifikatormasse der Probe, m_{B}, eine Modifikator-Molmasse der Probe, M_{B}, und mindestens einem UV-Extinktionskoeffizienten der Probe (152) ergibt;
Ausführen, durch das Computersystem, eines Satzes von logischen Operationen, die eine Kapsidproteinmasse der Probe, m_{A}, und eine Kapsidprotein-Molmasse der Probe, M_{A}, in Bezug auf mindestens einen Brechungsindexinkrementwert von dem mindestens einen Konzentrationsdetektor (154) berechnen;
Empfangen eines Injektionsvolumens der Probe, v, von einer Injektionsvolumendatenquelle (156); und
Ausführen, durch das Computersystem, eines Satzes von logischen Operationen, die eine absolute VGDV-Partikelkonzentration der Probe, C_{A}, berechnen, mittels ${C}_{A} = \left({m}_{A} x N\right) / \left({M}_{A} x v\right) ,$
wobei N die Avogadro-Zahl ist (158).

12. Computerimplementiertes Verfahren, umfassend:
Ausführen, durch ein Computersystem, eines Satzes von logischen Operationen, die eine Probe eines Virusgenabgabevehikels (VGDV) auf einem Satz von Analyseinstrumenten analysieren,
wobei der Satz mindestens ein Trenninstrument, mindestens ein statisches Lichtstreuinstrument und mindestens zwei Konzentrationsdetektoren umfasst (161);
Ausführen, durch das Computersystem, eines Satzes von logischen Operationen, die einen Massenanteil eines Proteins in der Probe, X_{A}, in Bezug auf einen aus der Probe bei einer ersten Wellenlänge, λ1, erfassten Ultraviolett-Absorptionswert, A^{λ1}, einen aus der Probe bei einer zweiten Wellenlänge, λ2, erfassten Ultraviolett-Absorptionswert, A^{λ2}, einen Extinktionskoeffizient des Proteins, ε_{A}^{λ1}, bei der ersten Wellenlänge, λ1, einen Extinktionskoeffizient des Proteins, ε_{A}^{λ2}, bei der zweiten Wellenlänge, λ2, einen Extinktionskoeffizient eines Modifikators in der Probe, ε_{B}^{λ1}, bei der ersten Wellenlänge, λ1, und einen Extinktionskoeffizient des Modifikators in der Probe, ε_{B}^{λ2}, bei der zweiten Wellenlänge, λ2, berechnen (162);
Ausführen, durch das Computersystem, eines Satzes von logischen Operationen, die einen Extinktionskoeffizienten der Probe bei der ersten Wellenlänge, ε_{VGDV}^{λ1}, in Bezug auf den Massenanteil des Proteins in der Probe, X_{A}, den Extinktionskoeffizienten des Proteins bei der ersten Wellenlänge, ε_{A}^{λ1}, und den Extinktionskoeffizienten des Modifikators in der Probe bei der ersten Wellenlänge, ε_{B}^{λ1}, berechnen (163);
Ausführen, durch das Computersystem, eines Satzes von logischen Operationen, die einen Extinktionskoeffizienten der Probe bei der zweiten Wellenlänge, ε_{VGDV}^{λ2}, in Bezug auf den Massenanteil des Proteins in der Probe, X_{A}, den Extinktionskoeffizienten des Proteins bei der zweiten Wellenlänge, ε_{A}^{λ2}, und den Extinktionskoeffizienten des Modifikators in der Probe bei der zweiten Wellenlänge, ε_{B}^{λ2}, berechnen (164);
Ausführen, durch das Computersystem, eines Satzes von logischen Operationen, die ein Brechungsindexinkrement der Probe, (dn/dc)_{VGDV}, in Bezug auf den Massenanteil des Proteins in der Probe, X_{A}, einen Brechungsindexkoeffizienten des Proteins, (dn/dc)_{A}, und einen Brechungsindexkoeffizienten des Modifikators in der Probe, (dn/dc)_{B}, berechnen (165),
Ausführen, durch das Computersystem, eines Satzes von logischen Operationen, die eine absolute Masse des Proteins, m_{A}, und eine absolute Masse des Modifikators, m_{B}, in Bezug auf einen aus der Probe bei einer Wellenlänge, λ, erfassten Ultraviolett-Absorptionswert, A^{λ}, den Massenanteil des Proteins in der Probe, X_{A}, einen Extinktionskoeffizienten des Proteins, ε_{A}^{λ} bei der Wellenlänge, λ, einen Extinktionskoeffizienten eines Modifikators in der Probe, ε_{B}^{λ,} bei der Wellenlänge, λ, wobei die Wellenlänge, λ, eine von der ersten Wellenlänge, λ1, und der zweiten Wellenlänge, λ₂, ist (166); und
Ausführen, durch das Computersystem, eines Satzes von logischen Operationen, die eine absolute VGDV-Partikelkonzentration der Probe, C_{A}, BERECHNEN, mittels ${C}_{A} = \left({m}_{A}\times N\right) / \left({M}_{A}\times v\right) ,$
wobei N die Avogadro-Zahl ist,
wobei M_{A} eine Kapsidprotein-Molmasse der Probe von einer Kapsidprotein-Molmassen-Datenquelle ist (167).

13. Verfahren nach Anspruch 12,
wobei das Berechnen des Massenanteils des Proteins in der Probe, X_{A}, das Berechnen des Massenanteils des Proteins in der Probe, X_{A}, umfasst mittels ${X}_{A} = \left(\left({A}^{λ 1}\times {{ε}_{B}}^{λ 2}\right)-\left({A}^{λ 2}\times {{ε}_{B}}^{λ 1}\right)\right) / \left(\left({A}^{λ 2}\times {{ε}_{A}}^{λ 1}\right)-\left({A}^{λ 2}\times {{ε}_{B}}^{λ 1}\right)-\left({A}^{λ 1}\times {{ε}_{A}}^{λ 2}\right)+\left({A}^{λ 1}\times \right.\right.$ ε_{B}^{λ2})), wobei das Berechnen des Extinktionskoeffizienten der Probe bei der ersten Wellenlänge ε_{VGDV}^{λ1}, das Berechnen des Extinktionskoeffizienten der Probe bei der ersten Wellenlänge, ε_{VGDV}^{λ1}, umfasst mittels ${{ε}_{VGDV}}^{λ 1} = \left({X}_{A}\times {{ε}_{A}}^{λ 1}\right) + \left(\left(1-{X}_{A}\right)\times {{ε}_{B}}^{λ 1}\right) ,$
wobei das Berechnen des Extinktionskoeffizienten der Probe bei der zweiten Wellenlänge, ε_{VGDV}^{λ2}, das Berechnen des Extinktionskoeffizienten der Probe bei der zweiten Wellenlänge, ε_{VGDV}^{λ2}, umfasst mittels ${{ε}_{VGDV}}^{λ 2} = \left({X}_{A}\times {{ε}_{A}}^{λ 2}\right) + \left(\left(1-{X}_{A}\right)\times {{ε}_{B}}^{λ 2}\right) ,$
wobei das Berechnen des Brechungsindexinkrements der Probe, (dn/dc)_{VGDV}, das Berechnen des Brechungsindexinkrements der Probe, (dn/dc)_{VGDV}, umfasst mittels ${\left(dn/dc\right)}_{VGDV} = \left({X}_{A}\times {\left(dn/dc\right)}_{A}\right) + \left(\left(1-{X}_{A}\right)\times {\left(dn/dc\right)}_{B}\right) ,$
wobei das Berechnen der absoluten Masse des Proteins, m_{A}, das Berechnen der absoluten Masse des Proteins, m_{A}, umfasst mittels ${m}_{A} = \left({A}^{λ}\times {X}_{A}\right) / \left(\left({X}_{A}\times {{ε}_{A}}^{λ}\right)+\left(\left(1-{X}_{A}\right)\times {{ε}_{B}}^{λ}\right)\right. ,$
wobei das Berechnen der absoluten Masse des Modifikators, m_{B}, das Berechnen der absoluten Masse des Modifikators, m_{B}, umfasst mittels ${m}_{B} = \left({A}^{λ}\times \left(1-{X}_{A}\right)\right) / \left(\left({X}_{A}\times {{ε}_{A}}^{λ}\right)+\left(\left(1-{X}_{A}\right)\times {{ε}_{B}}^{λ}\right)\right) .$

## Revendications

1. Procédé implémenté par ordinateur comprenant :
l'exécution, par un système d'ordinateur, d'un ensemble d'opérations logiques analysant un échantillon de véhicule d'apport de gène viral (VGDV) sur un ensemble d'instruments analytiques,
dans lequel l'ensemble comprend au moins un instrument de séparation, au moins un instrument de diffusion de lumière statique, et au moins deux détecteurs de concentration,
résultant en une masse de protéine de capside de l'échantillon, m_{A}, une masse de modificateur de l'échantillon, m_{B}, et une masse molaire de modificateur de l'échantillon, M_{B} (110) ;
la réception d'une masse molaire de protéine de capside de l'échantillon, M_{A}, à partir d'une source de données de masse molaire de protéine de capside (112) ;
la réception d'un volume d'injection de l'échantillon, v, à partir d'une source de données de volume d'injection (114), et
l'exécution, par le système d'ordinateur, d'un ensemble d'opérations logiques calculant une concentration particulaire de VGDV totale de l'échantillon, C_{A}, par l'intermédiaire de ${C}_{A} = \left({m}_{A}\times N\right) / \left({M}_{A}\times v\right) ,$
dans lequel N est le nombre d'Avogadro (116).

2. Procédé selon la revendication 1 comprenant en outre :
l'exécution, par le système d'ordinateur, d'un ensemble d'opérations logiques analysant l'échantillon sur l'ensemble, résultant en la masse molaire de protéine de capside de l'échantillon, M_{A} ; et
le stockage de la masse molaire de protéine de capside de l'échantillon, M_{A}, dans la source de données de masse molaire de protéine de capside.

3. Procédé selon la revendication 1 dans lequel l'analyse comprend l'analyse de l'échantillon sur l'ensemble par l'intermédiaire d'une technique d'analyse,
dans lequel la technique d'analyse est l'une parmi une analyse de vecteur viral, une analyse de conjugué de protéines, et une analyse de composition de copolymère.

4. Procédé selon la revendication 1 dans lequel les au moins deux détecteurs de concentration comprennent un premier détecteur d'absorbance d'ultraviolet à une première longueur d'onde, λ1, et un second détecteur d'absorbance d'ultraviolet à une seconde longueur d'onde, λ2.

5. Procédé selon la revendication 1 dans lequel les au moins deux détecteurs de concentration comprennent un détecteur d'absorbance d'ultraviolet à une longueur d'onde, λ, et un détecteur d'indice de réfraction différentiel.

6. Procédé selon la revendication 1 dans lequel les au moins deux détecteurs de concentration comprennent un détecteur d'absorbance d'ultraviolet à une longueur d'onde, λ, et un détecteur de fluorescence.

7. Procédé selon la revendication 1 comprenant en outre :
la réception, par le système d'ordinateur, d'une masse molaire d'un modificateur complet à l'intérieur d'un échantillon de VGDV complet, M_{Full}, à partir d'une source de données de masse molaire de modificateur complet ;
l'exécution, par le système d'ordinateur, d'un ensemble d'opérations logiques calculant une concentration de VGDV complet de l'échantillon de VGDV complet, C_{Full}, par l'intermédiaire de ${C}_{Full} = \left({m}_{B}\times N\right) / \left({M}_{Full}\times v\right) ;$ et
l'exécution, par le système d'ordinateur, d'un ensemble d'opérations logiques calculant une concentration de VGDV vide de l'échantillon de VGDV complet, C_{Empty}, par l'intermédiaire de ${C}_{Empty} = {C}_{A} - {C}_{Full} .$

8. Procédé selon la revendication 7 comprenant en outre
l'exécution, par le système d'ordinateur, d'un ensemble d'opérations logiques analysant l'échantillon de VGDV complet sur l'ensemble,
résultant en la masse molaire du modificateur complet à l'intérieur de l'échantillon de VGDV complet, M_{Full} ; et
le stockage de la masse molaire du modificateur complet à l'intérieur de l'échantillon de VGDV complet, M_{Full}, dans la source de données de masse molaire de modificateur complet.

9. Procédé selon la revendication 1 comprenant en outre :
l'exécution, par le système d'ordinateur, d'un ensemble d'opérations logiques analysant une région de signal de VGDV entière de l'échantillon sur l'ensemble et analysant une région de pic agrégé de l'échantillon sur l'ensemble,
résultant en une masse de protéine de pic entier de VGDV de l'échantillon, m_{A}, ₑₙₜ, correspondant à la région de signal de VGDV entière de l'échantillon, une masse de modificateur de pic entier de VGDV de l'échantillon, m_{B}, ₑₙₜ, correspondant à la région de signal de VGDV entière de l'échantillon, une masse molaire de protéine de pic entier de VGDV de l'échantillon, M_{A}, ₑₙₜ, correspondant à la région de signal de VGDV entière de l'échantillon, une masse molaire de modificateur de pic entier de VGDV de l'échantillon, M_{B}, ₑₙₜ, correspondant à la région de signal de VGDV entière de l'échantillon, une masse de protéine de pic agrégé de VGDV de l'échantillon, m_{A}, _{agg}, correspondant à la région de pic agrégé de l'échantillon, une masse de modificateur de pic agrégé de VGDV de l'échantillon, m_{B}, _{agg}, correspondant à la région de pic agrégé de l'échantillon, une masse molaire de protéine de pic agrégé de VGDV de l'échantillon, M_{A}, _{agg}, correspondant à la région de pic agrégé de l'échantillon, une masse molaire de modificateur de pic agrégé de VGDV de l'échantillon, M_{B}, _{agg}, correspondant à la région de pic agrégé de l'échantillon ; et
l'exécution, par le système d'ordinateur, d'un ensemble d'opérations logiques calculant une concentration particulaire de pic entier de VGDV totale de l'échantillon, C_{A}, ₑₙₜ, correspondant à la région de signal de VGDV ent
ière de l'échantillon, par l'intermédiaire de ${C}_{A , ent} = \left({m}_{A , ent} x N\right) / \left({M}_{A , ent} x v\right) ;$ et
l'exécution, par le système d'ordinateur, d'un ensemble d'opérations logiques calculant une concentration particulaire de pic agrégé de VGDV totale de l'échantillon, C_{A}, _{AGG}, correspondant à la région de pic agrégé de l'échantillon, par l'intermédiaire de ${C}_{A , agg} = \left({m}_{A , agg} x N\right) / \left({M}_{A , agg} x v\right) .$

10. Procédé selon la revendication 9 comprenant en outre :
l'exécution, par le système d'ordinateur, d'un ensemble d'opérations logiques analysant une région de signal de VGDV entière d'un échantillon de véhicule d'apport de gène viral complet sur l'ensemble et analysant une région de pic agrégé de l'échantillon complet sur l'ensemble,
résultant en une masse molaire de pic entier de VGDV d'un modificateur complet à l'intérieur de l'échantillon complet, M_{Full}, ₑₙₜ, correspondant à la région de signal de VGDV entière de l'échantillon complet, et une masse molaire de pic agrégé de VGDV du modificateur complet à l'intérieur de l'échantillon complet, M_{Full}, _{agg}, correspondant à la région de pic agrégé de l'échantillon complet ;
l'exécution, par le système d'ordinateur, d'un ensemble d'opérations logiques calculant une concentration de VGDV complet de pic entier de VGDV de l'échantillon complet, C_{FULL}, ₑₙₜ, correspondant à la région de signal de VGDV entière de l'échantillon de VGDV, par l'intermédiaire de ${C}_{Full , ent} = \left({m}_{B , ent} x N\right) / \left({M}_{Full , ent} x v\right) ;$
l'exécution, par le système d'ordinateur, d'un ensemble d'opérations logiques calculant une concentration de VGDV complet de pic agrégé de VGDV de l'échantillon complet, C_{FULL}, _{AGG}, correspondant à la région de pic agrégé de l'échantillon complet, par l'intermédiaire de ${C}_{Full , agg} = \left({m}_{B , agg} x N\right) / \left({M}_{Full , agg} x v\right)$
l'exécution, par le système d'ordinateur, d'un ensemble d'opérations logiques calculant une concentration de VGDV vide de pic entier de VGDV de l'échantillon complet, C_{Empty}, ₑₙₜ, correspondant à la région de signal de VGDV entière de l'échantillon complet, par l'intermédiaire de ${C}_{Empty , ent} = {C}_{A , ent} - {C}_{Full , ent} ;$ et
l'exécution, par le système d'ordinateur, d'un ensemble d'opérations logiques calculant une concentration de VGDV vide de pic agrégé de VGDV de l'échantillon complet, C_{Empty}, _{agg}, correspondant à la région de pic agrégé de l'échantillon complet, par l'intermédiaire de ${C}_{Empty , agg} = {C}_{A , agg} - {C}_{Full , agg} .$

11. Procédé implémenté par ordinateur comprenant :
l'exécution, par un système d'ordinateur, d'un ensemble d'opérations logiques analysant un échantillon de véhicule d'apport de gène viral (VGDV) sur un ensemble d'instruments analytiques,
dans lequel l'ensemble comprend au moins un instrument de séparation, au moins un instrument de diffusion de lumière statique, et au moins un détecteur de concentration,
résultant en une masse de modificateur de l'échantillon, m_{B}, une masse molaire de modificateur de l'échantillon, M_{B}, et au moins un coefficient d'extinction UV de l'échantillon (152) ;
l'exécution, par le système d'ordinateur, d'un ensemble d'opérations logiques calculant une masse de protéine de capside de l'échantillon, m_{A}, et une masse molaire de protéine de capside de l'échantillon, M_{A}, par rapport à au moins une valeur d'incrément d'indice de réfraction provenant de l'au moins un détecteur de concentration (154) ;
la réception d'un volume d'injection de l'échantillon, v, à partir d'une source de données de volume d'injection (156) ; et
l'exécution, par le système d'ordinateur, d'un ensemble d'opérations logiques calculant une concentration particulaire de VGDV totale de l'échantillon, C_{A}, par l'intermédiaire de ${C}_{A} = \left({m}_{A} x N\right) / \left({M}_{A} x v\right) ,$
dans lequel N est le nombre d'Avogadro (158).

12. Procédé implémenté par ordinateur comprenant :
l'exécution, par un système d'ordinateur, d'un ensemble d'opérations logiques analysant un échantillon de véhicule d'apport de gène viral (VGDV) sur un ensemble d'instruments analytiques,
dans lequel l'ensemble comprend au moins un instrument de séparation, au moins un instrument de diffusion de lumière statique, et au moins deux détecteurs de concentration (161) ;
l'exécution, par le système d'ordinateur, d'un ensemble d'opérations logiques calculant une fraction massique d'une protéine dans l'échantillon, X_{A}, par rapport à une valeur d'absorbance d'ultraviolet, A^{λ1}, recueillie à partir de l'échantillon à une première longueur d'onde, λ1, à une valeur d'absorbance d'ultraviolet, A^{λ2}, recueillie à partir de l'échantillon à une seconde longueur d'onde, λ2, à un coefficient d'extinction de la protéine, ε_{A}^{λ1}, à la première longueur d'onde, λ1, à un coefficient d'extinction de la protéine, ε_{A}^{λ2}, à la seconde longueur d'onde, λ2, à un coefficient d'extinction d'un modificateur dans l'échantillon, ε_{B}^{λ1}, à la première longueur d'onde, λ1, et à un coefficient d'extinction du modificateur dans l'échantillon, ε_{B}^{λ2}, à la seconde longueur d'onde, λ2 (162) ;
l'exécution, par le système d'ordinateur, d'un ensemble d'opérations logiques calculant un coefficient d'extinction de l'échantillon à la première longueur d'onde, ε_{VGDV}^{λ1}, par rapport à la fraction massique de la protéine dans l'échantillon, X_{A}, au coefficient d'extinction de la protéine à la première longueur d'onde, ε_{A}^{λ1}, et au coefficient d'extinction du modificateur dans l'échantillon à la première longueur d'onde, ε_{B}^{λ1} (163) ;
l'exécution, par le système d'ordinateur, d'un ensemble d'opérations logiques calculant un coefficient d'extinction de l'échantillon à la seconde longueur d'onde, ε_{VGDV}^{λ2}, par rapport à la fraction massique de la protéine dans l'échantillon, X_{A}, au coefficient d'extinction de la protéine à la seconde longueur d'onde, ε_{A}^{λ2}, et au coefficient d'extinction du modificateur dans l'échantillon à la seconde longueur d'onde, ε_{B}^{λ2} (164) ;
l'exécution, par le système d'ordinateur, d'un ensemble d'opérations logiques calculant un incrément d'indice de réfraction de l'échantillon, (dn/dc)_{VGDV}, par rapport à la fraction massique de la protéine dans l'échantillon, X_{A}, à un coefficient d'indice de réfraction de la protéine, (dn/dc)_{A}, et à un coefficient d'indice de réfraction du modificateur dans l'échantillon, (dn/dc)_{B} (165),
l'exécution, par le système d'ordinateur, d'un ensemble d'opérations logiques calculant une masse totale de la protéine, m_{A}, et une masse totale du modificateur, m_{B}, par rapport à une valeur d'absorbance d'ultraviolet, A^{λ}, recueillie à partir de l'échantillon à une longueur d'onde, λ, à la fraction massique de la protéine dans l'échantillon, X_{A}, à un coefficient d'extinction de la protéine, ε_{A}^{λ} à la longueur d'onde, λ, à un coefficient d'extinction d'un modificateur dans l'échantillon, e_{B}^{λ}, à la longueur d'onde, λ, où la longueur d'onde, λ, est l'une parmi la première longueur d'onde, λ1, et la seconde longueur d'onde, λ₂ (166) ; et
l'exécution, par le système d'ordinateur, d'un ensemble d'opérations logiques calculant une concentration particulaire de VGDV totale de l'échantillon, C_{A}, par l'intermédiaire de ${C}_{A} = \left({m}_{A} x N\right) / \left({M}_{A} x v\right) ,$
dans lequel N est le nombre d'Avogadro,
dans lequel M_{A} est une masse molaire de protéine de capside de l'échantillon provenant d'une source de données de masse molaire de protéine de capside (167).

13. Procédé selon la revendication 12,
dans lequel le calcul de la fraction massique de la protéine dans l'échantillon, X_{A}, comprend le calcul de la fraction massique de la protéine dans l'échantillon, X_{A}, par l'intermédiaire de ${\text{X}}_{\text{A}} = \left(\left({\text{A}}^{λ 1} {\text{x}}_{\text{B}} {}^{λ \text{2}}\right) - \left({\text{A}}^{λ 2} \text{x} {ε}_{\text{B}} {}^{λ \text{1}}\right)\right) / \left(\left({\text{A}}^{λ \text{2}} \text{x} {ε}_{\text{A}} {}^{λ \text{1}}\right) - \left({\text{A}}^{λ \text{2}} \text{x} {ε}_{\text{B}} {}^{λ \text{1}}\right) - \left({\text{A}}^{λ \text{1}} \text{x} {ε}_{\text{A}} {}^{λ \text{2}}\right) + \left({\text{A}}^{λ \text{1}} \text{x} {ε}_{\text{B}} {}^{λ \text{2}}\right)\right) \text{,}$
dans lequel le calcul du coefficient d'extinction de l'échantillon à la première longueur d'onde, ε_{VGDV}^{λ1}, comprend le calcul du coefficient d'extinction de l'échantillon à la première longueur d'onde, ε_{VGDV}^{λ1}, par l'intermédiaire de ${{ε}_{VGDV}}^{λ 1} = \left({X}_{A} x {{ε}_{A}}^{λ 1}\right) + \left(\left(1-{X}_{A}\right) x {{ε}_{B}}^{λ 1}\right) ,$
dans lequel le calcul du coefficient d'extinction de l'échantillon à la seconde longueur d'onde, ε_{VGDV}^{λ2}, comprend le calcul du coefficient d'extinction de l'échantillon à la seconde longueur d'onde, ε_{VGDV}^{λ2}, par l'intermédiaire de ${{ε}_{VGDV}}^{λ 2} = \left({X}_{A} x {{ε}_{A}}^{λ 2}\right) + \left(\left(1-{X}_{A}\right) x {{ε}_{B}}^{λ 2}\right) ,$
dans lequel le calcul de l'incrément d'indice de réfraction de l'échantillon, (dn/dc)_{VGDV}, comprend le calcul de l'incrément d'indice de réfraction de l'échantillon, (dn/dc)_{VGDV}, par l'intermédiaire de ${\left(dn/dc\right)}_{VGDV} = \left({X}_{A} x {\left(dn/dc\right)}_{A}\right) + \left(\left(1-{X}_{A}\right) x {\left(dn/dc\right)}_{B}\right) ,$
dans lequel le calcul de la masse totale de la protéine, m_{A}, comprend le calcul de la masse totale de la protéine, m_{A}, par l'intermédiaire de ${m}_{A} = \left({A}^{λ} x {X}_{A}\right) / \left(\left({X}_{A} x {{ε}_{A}}^{λ}\right)+\left(\left(1-{X}_{A}\right) x {{ε}_{B}}^{λ}\right)\right) ,$
dans lequel le calcul de la masse totale du modificateur, m_{B}, comprend le calcul de la masse totale du modificateur, m_{B}, par l'intermédiaire de ${m}_{B} = \left({A}^{λ} x \left(1-{X}_{A}\right)\right) / \left(\left({X}_{A} x {{ε}_{A}}^{λ}\right)+\left(\left(1-{X}_{A}\right) x {{ε}_{B}}^{λ}\right)\right) .$
